# EUROPEAN PATENT APPLICATION

(11) **EP 2 554 514 A1**
(43) Date of publication of application: **06.02.2013**
(21) Application number: 11765723.9
(22) Date of filing: 31.03.2011
(51) Int. Cl.: C01B 15/01, A61L 2/20

(54) **HYDROGEN PEROXIDE GAS PRODUCTION DEVICE**

(30) Priority: 31.03.2010 JP 2010083621; 31.03.2010 JP 2010083620; 31.03.2010 JP 2010083619
(71) Applicant: Panasonic Healthcare Co., Ltd., Ehime 791-0395 (JP)
(72) Inventor: FUKUI, Shinji, Chuo-ku Osaka 540-6207 (JP); YOKOI, Yasuhiko, Chuo-ku Osaka 540-6207 (JP); OHNISHI, Jirou, Chuo-ku Osaka 540-6207 (JP); IWAMA, Akifumi, Chuo-ku Osaka 540-6207 (JP)
(74) Representative: Grünecker, Kinkeldey, Stockmair & Schwanhäusser
(86) International application number: PCT/JP2011/058182
(87) International publication number: WO 2011/125823

(57) **Abstract**

In order to reduce a, remaining solution when hydrogen peroxide gas is produced to a minimum, disclosed is a hydrogen peroxide gas production device characterized by being provided with an atomization unit which atomizes hydrogen peroxide stored in a storage part by applying ultrasonic vibration to the hydrogen peroxide, a heater which is provided above the atomization unit and heats the hydrogen peroxide atomized by the atomization unit to gasify the hydrogen peroxide, a metallic internal cylinder part in which the heater is disposed in the inner space thereof and which guides upward the hydrogen peroxide that is atomized by the atomization unit and flows together with carrier gas, and an external cylinder part in which the internal cylinder part is disposed in the inner space thereof to configure a double tube and which forms, with the internal cylinder part, a gas flow path for the carrier gas that goes down toward the storage part therebetween, and characterized by being configured so that the carrier gas flowing through the gas flow path is brought into contact with the internal cylinder part heated by the heater and the heated carrier gas is introduced into the storage part.

## Description

### FIELD OF THE INVENTION

The present invention relates to a hydrogen peroxide gas generator.

### BACKGROUND OF THE INVENTION

An isolator is a device including an aseptic working chamber in an interior thereof. In this working chamber, a work requiring an aseptic environment is performed. For example, a work such as cell culturing is performed. In this isolator, the interior of the working chamber is sterilized by spraying a gasified hydrogen peroxide solution into the working chamber (See Patent Documents 1 and 2).

A sterilization gas generator configured to generate a sterilizing gas such as a hydrogen peroxide gas and the like is generally provided with an atomizing device configured to atomize the hydrogen peroxide solution stored in a storage portion by applying supersonic vibration. Moreover, the sterilization gas generator generates a hydrogen peroxide gas by heating atomized hydrogen peroxide solution (See Patent Document 3).

Sterilization means killing microorganisms establishing the highest degree of aseptic condition possible, however, in the present specification, it is assumed that sterilization includes so-called decontamination, decolonization, disinfection and the like.

Moreover, an aseptic environment means an aseptic environment to the highest degree possible, decontamination processing means processing for realizing the aseptic environment, and substances used for the decontamination processing are referred to as decontamination substances.

### PRIOR ART DOCUMENTS

### PATENT DOCUMENTS

Patent Document 1: Japanese Patent Application Laid-open Publication No. 2006-320392
Patent Document 2: Japanese Patent Application Laid-open Publication No. 2005-312799
Patent Document 3: Japanese Patent Application Laid-open Publication No. 2005-172692

### SUMMARY OF THE INVENTION

### Problems to be solved by the invention

Hydrogen peroxide has strong sterilizing power and corroding power, and gives a strong stimulus when a solution with high concentration adheres to the skin of a human being. Thus, in gasifying the hydrogen peroxide solution, it is required to reduce a remaining solution to the smallest amount possible.

Moreover, if the sterilization gas generator atomizes the hydrogen peroxide solution, an amount of the hydrogen peroxide solution in the storage portion decreases, and the concentration of the hydrogen peroxide solution increases. Since the hydrogen peroxide solution with high concentration is difficult to be gasified, there is a risk that the hydrogen peroxide solution with high concentration might remain in the storage portion.

Moreover, a vibration plate provided in the storage portion so as to atomize the hydrogen peroxide solution vibrates upon receipt of ultrasonic waves transmitted through transmitting water. Such a vibration plate is extremely thin in general, thus a crack or the like might appear in the vibration plate and the transmitting water might intrude into the storage portion. Therefore, in the sterilization gas generator, some liquid may be present in the storage portion even before the hydrogen peroxide solution is supplied, for example,

The present invention has been made considering such circumstances, and the object thereof is to reduce the residual solution to the smallest amount possible when generating a hydrogen peroxide gas.

Moreover, another object is to provide a sterilization substance generator in which hydrogen peroxide solution with high concentration cannot easily remain in a storage portion.

Furthermore, another object is to provide an atomizing device capable of detecting whether or not a liquid remains in a storage portion.

### Means of solving the problems

A hydrogen peroxide gas generator according to an aspect of the present invention, includes: an atomizing unit configured to atomize hydrogen peroxide stored in a storage portion by applying ultrasonic vibration; a heater provided above the atomizing unit, the heater configured to heat and gasify the hydrogen peroxide atomized in the atomizing unit; an internal cylindrical portion, made of metal, whose internal space has the heater arranged therein, the internal cylindrical portion configured to guide upward the hydrogen peroxide atomized in the atomizing unit flowing together with a carrier gas; and an external cylindrical portion, double-pipe constructed, whose internal space has the internal cylindrical portion arranged therein, having a gas flow path for the carrier gas flowing downward toward the storage portion formed between the external cylindrical portion and the internal cylindrical portion, the carrier gas flowing through the gas flow path allowed to contact the internal cylindrical portion heated by the heater, the heated carrier gas introduced to the storage portion.

Moreover, a sterilization substance generator according to another aspect of the present invention, includes: an atomizing unit including a storage portion configured to store hydrogen peroxide solution, and an ultrasonic vibrator configured to atomize the hydrogen peroxide solution by applying ultrasonic vibration to the hydrogen peroxide solution stored in the storage portion; a gasifying unit configured to heat and gasify the hydrogen peroxide solution atomized by the atomizing unit, and output the gasified solution together with a supplied carrier gas; a first supply unit configured to supply the hydrogen peroxide solution to the storage portion; a second supply unit configured to supply a diluent to the storage portion; a determination unit configured to determine whether or not an amount of the hydrogen peroxide solution remaining in the storage portion has reached a first predetermined amount since atomization of the hydrogen peroxide solution stored in the storage portion, based on a supply amount of the hydrogen peroxide solution supplied from the first supply unit to the storage portion; and a control unit configured to control the atomizing unit so as to atomize the hydrogen peroxide solution stored in the storage portion, and control the second supply unit so as to supply the diluent to the storage portion when the determination unit has determined that an amount of the hydrogen peroxide solution remaining in the storage portion has reached the first predetermined amount.

Furthermore, an atomizing device according to another aspect of the present invention, includes: a first storage portion configured to store transmitting water; a second storage portion, configured to store liquid, provided such that a bottom surface attached with a vibration plate is immersed in the transmitting water; an ultrasonic vibrator configured to apply ultrasonic vibration to the vibration plate through the transmitting water and atomize the liquid, the ultrasonic vibrator mounted on a bottom surface of the first storage portion such that a face of the ultrasonic vibrator where ultrasonic waves are generated forms a predetermined angle with respect to a horizontal direction; a driving unit configured to drive the ultrasonic vibrator so as to generate ultrasonic waves at the ultrasonic vibrator; a measuring unit configured to measure a current supplied to the driving unit from a power supply supplied to the driving unit; and a determination unit configured to determine whether or not the liquid remains in the second storage portion or the transmitting water has intruded into the second storage portion through the vibration plate, based on a measurement result of the measuring unit.

### Advantageous effect of the invention

With a hydrogen peroxide gas generator according to the present invention, since a carrier gas heated by an internal cylindrical portion is introduced into a storage portion, hydrogen peroxide solution in the storage portion is evaporated by heat exchange with the carrier gas. Thus, it is possible to reduce the hydrogen peroxide solution remaining in the storage portion to the smallest amount possible.

Moreover, with a sterilization substance generator according to another aspect of the present invention, it is possible to provide a sterilization substance generator in which hydrogen peroxide solution with high concentration cannot easily remain in a storage portion.

Furthermore, with an atomizing device according to another aspect of the present invention, it is possible to provide an atomizing device capable of detecting whether or not a liquid remains in a storage portion.

### BRIEF DESCRIPTION OF THE DRAWINGS

Fig. 1 is a diagram illustrating a configuration of an isolator.
Fig. 2 is a diagram illustrating a configuration of a sterilization gas generator according to a first embodiment of the present invention.
Fig. 3 is an enlarged view of a periphery of a storage portion illustrating a periphery of an ultrasonic vibrator in an exploded manner.
Fig. 4 is a graph for explaining a change over time in temperature at each unit.
Fig. 5 is a diagram illustrating a configuration of a sterilization gas generator according to a comparative example.
Fig. 6 is a graph for explaining a change over time in temperature at each unit.
Fig. 7 is a diagram illustrating a configuration of a sterilization gas generator according to a second embodiment of the present invention.
Fig. 8 is an enlarged view of a periphery of a storage portion illustrating a periphery of an ultrasonic vibrator in an exploded manner.
Fig. 9 is a diagram illustrating a configuration of a sterilization gas generator according to a third embodiment of the present invention.
Fig. 10 is a diagram illustrating a configuration of a sterilization gas generator according to a fourth embodiment of the present invention.
Fig. 11 is a diagram illustrating a configuration of an isolator 1010 according to an embodiment of the present invention.
Fig. 12 is a side view of a sterilization gas generator 1035.
Fig. 13 is a diagram illustrating a functional block realized in a microcomputer 1071.
Fig. 14 is a flowchart illustrating an example of processing executed by a microcomputer 1071.
Fig. 15 is a flowchart illustrating an example of processing executed by a microcomputer 1071.
Fig. 16 is a diagram for explaining an operation when a sterilization gas generator 1035 generates a hydrogen peroxide gas.
Fig. 17 is a diagram illustrating a configuration of an isolator 2010 according to an embodiment of the present invention.
Fig. 18 is a side view of a sterilization gas generator 2035.
Fig. 19 is a diagram illustrating an example of a measured waveform of a current IA when an ultrasonic vibrator 2121 is operating in a case where there is water in a cup 2100 and a case where there is not.
Fig. 20 is a diagram illustrating an example of an addition result of an absolute value of fluctuation of a current IA in a case where there is water in a cup 2100 and a case where there is not.
Fig. 21 is a diagram illustrating a functional block realized by a microcomputer 2074.
Fig. 22 is a flowchart illustrating an example of processing executed by a microcomputer 2074.

### MODES FOR CARRYING OUT THE INVENTION

### Example 1

An example 1 of the present invention will be described below. A hydrogen peroxide gas generator according to the present invention is incorporated in an isolator as a sterilization gas generator according to an embodiment of the present invention. Thus, description will be given by using an isolator as an example.

### <<First embodiment>>

### <Entire configuration of isolator>

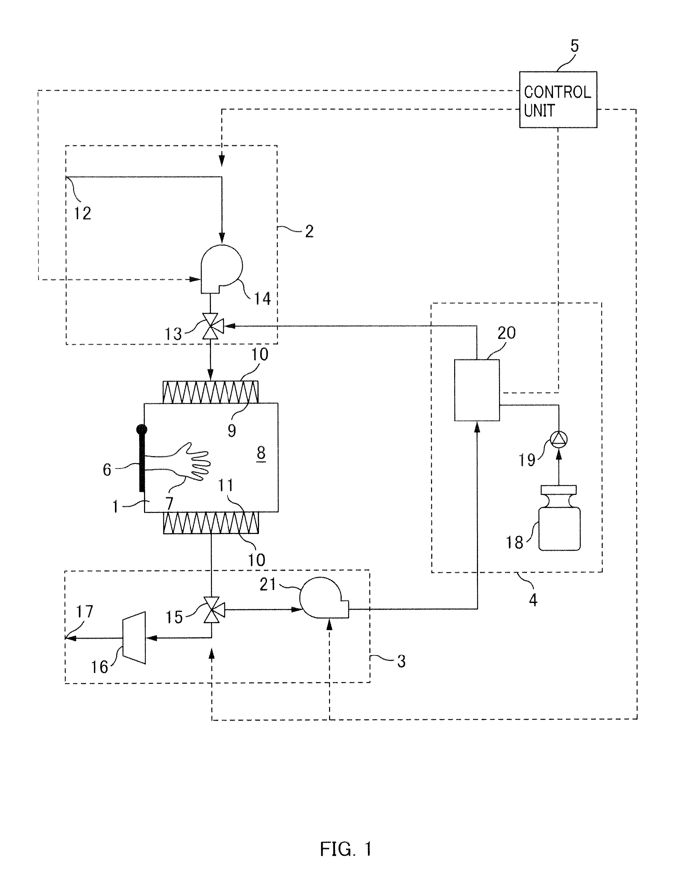

As illustrated in Fig. 1, an isolator includes a working chamber 1, a gas supply unit 2, a gas discharge unit 3, a sterilizing gas supply device 4, and a control unit 5.

The working chamber 1 is a portion defining a working space for performing a work in an aseptic environment, and is configured with a box-shaped member having a front-surface door 6 on the front surface. The front surface door 6 is formed in such a manner as to be capable of being opened/closed from an exterior. In this front-surface door 6, a working glove 7 is provided. An arm of a worker is inserted into this working glove 7 when working in a working space 8. A gas supply port 9 is provided in one of side surfaces in the working chamber 1. A gas (hydrogen peroxide gas for sterilization, for example) is supplied from a gas supply unit 2 through this gas supply port 9. Here, the gas supply port 9 is provided with an HEPA filter 10. Thus, dusts and the like contained in the gas from the gas supply unit 2 is caught by the HEPA filter 10, and only the gas is supplied to the working space 8. A gas discharge port 11 is provided in the other side surface in the working chamber 1. This gas discharge port 11 is also provided with HEPA filter 10. Thus, entry of dusts and the like into the working space 8 through the gas discharge port 11 is prevented. The gas in the working space 8 is discharged through the gas discharge port 11. The discharged gas is sent to the gas discharge unit 3.

The gas supply unit 2 is a portion configured to supply a gas to the working chamber 1. This gas supply unit 2 is provided with an air inlet 12, a first three-way valve 13, and a fan 14. The air inlet 12 is a portion configured to take in air from the exterior. The fan 14 is configured to send out the air taken in from the exterior to the first three-way valve 13.

The first three-way valve 13 is communicated with each of the sterilizing gas supply device 4, the fan 14, and the working chamber 1. Flow paths are switched in accordance with control information from the control unit 5.

Therefore, when the sterilizing gas supply device 4 is communicated with the working chamber 1 by the first three-way valve 13, the hydrogen peroxide gas is supplied to the working chamber 1.

On the other hand, if the fan 14 is communicated with the working chamber 1 by the first three-way valve 13, the air is supplied to the working chamber 1 by an operation of the fan 14. The fan 14 is switched between an operation state and a stop state by a control signal from the control unit 5. Further, a sending-out amount of a gas can be also adjusted.

The gas discharge unit 3 is a portion configured to discharge a gas into the working chamber 1. This gas discharge unit 3 is provided with a second three-way valve 15, a sterilizing substance reduction processing unit 16, an air outlet 17, and a fan 21. The second three-way valve 15 is communicated with each of the gas discharge port 11 of the working chamber 1, the fan 21, and the sterilizing substance reduction processing unit 16. Flow paths are switched in accordance with control information from the control unit 5. For example, the gas discharge port 11 is communicated with the sterilizing substance reduction processing unit 16 or the gas discharge port 11 is communicated with the fan 21.

The fan 21 supplies a gas from the gas discharge port 11 to a sterilization gas generator 20 as a so-called carrier gas. Thus, when the gas discharge port 11 is communicated with the fan 21, and the sterilization gas generator 20 is communicated with the working chamber 1, the gas from the gas discharge port 11 circulates in the isolator.

The sterilizing substance reduction processing unit 16 is provided between the second three-way valve 15 and the air outlet 17, and is a portion configured to perform processing for reducing the concentration of hydrogen peroxide (sterilizing substance) in the gas sent through the second three-way valve 15. This sterilizing substance reduction processing unit 16 is configured with a metal catalyst such as platinum or an active coal, for example. The sterilizing substance reduction processing unit 16 is not limited to the metal catalyst or active coal, as long as the concentration of hydrogen peroxide can be reduced. The air outlet 17 is a portion for discharging the gas, having been processed in the sterilizing substance reduction processing unit 16, into the atmosphere.

The sterilizing gas supply device 4 is a portion configured to gasify hydrogen peroxide, to be supplied therefrom and corresponds to the hydrogen peroxide supply device. This sterilizing gas supply device 4 includes a sterilizing substance cartridge 18, a pump 19, and the sterilization gas generator 20. The sterilizing substance cartridge 18 is configured to store a hydrogen peroxide solution. The pump 19 is configured to pump up the hydrogen peroxide solution stored in the sterilizing substance cartridge 18 and send this out to the sterilization gas generator 20. This pump 19 is configured with a peristaltic pump, for example. The sterilization gas generator 20 is configured to generate a hydrogen peroxide gas from the supplied hydrogen peroxide solution. The generated hydrogen peroxide gas is supplied to the first three-way valve 13, for example. The sterilization gas generator 20 will be described later in detail.

The control unit 5 is a portion configured to electrically control each of these portions. This control unit 5 controls the first three-way valve 13 and the fan 14 included in the gas supply unit 2, the second three-way valve 15 included in the gas discharge unit 3, the sterilization gas generator 20 included in the sterilizing gas supply device 4, and the like. For example, the hydrogen peroxide gas generated by the sterilization gas generator 20 can be supplied to the working chamber 1 through the first three-way valve 13. Further, the hydrogen peroxide gas supplied to the working chamber 1 can be sent to the first three-way valve 13 side through the second three-way valve 15, thereby being able to circulate it in a system. If the hydrogen peroxide gas is circulated in the system, an aseptic environment can be established in the interior of the system. Here, the aseptic environment refers to a dust-free and/or aseptic environment to the highest degree possible, where a substance other than that required for the work performed in the working chamber 1 is prevented from intruding.

### <Sterilization gas generator 20>

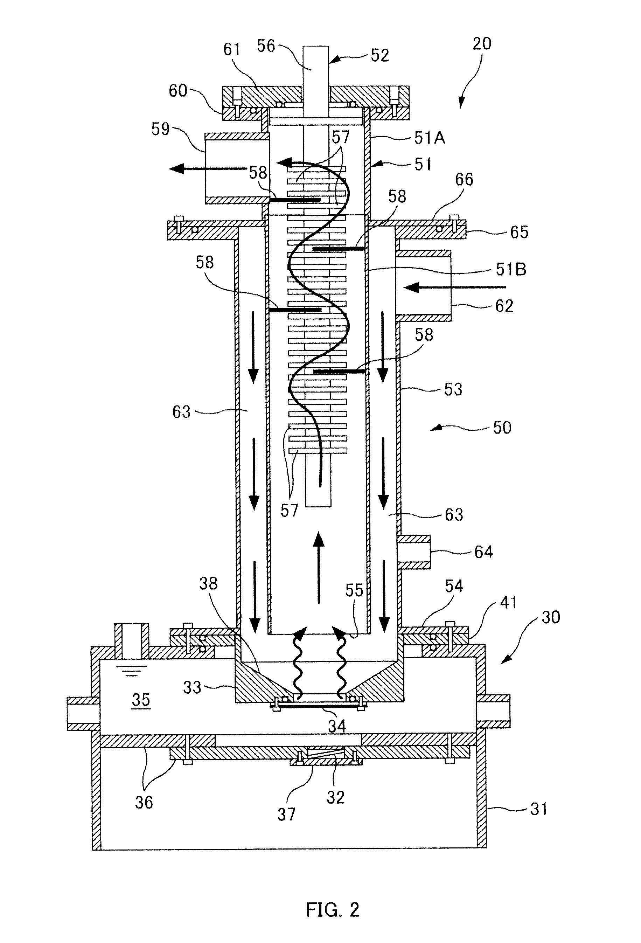

Subsequently, the sterilization gas generator 20 will be described. This sterilization gas generator 20 corresponds to a hydrogen peroxide gas generator. As illustrated in Fig. 2, the sterilization gas generator 20 includes an atomizing unit 30 and a gasifying unit 50. The atomizing unit 30 is a portion configured to atomize the hydrogen peroxide solution supplied from the pump 19, and the gasifying unit 50 is a portion configured to gasify the atomized hydrogen peroxide.

First, the atomizing unit 30 will be described. The atomizing unit 30 includes a housing unit 31, an ultrasonic vibrator 32, a storage portion 33, and a vibration plate 34, and the like.

The housing unit 31 is a portion configured to house the ultrasonic vibrator 32 and an ultrasonic transmitting liquid 35, and is a hollow cylindrical container having an opening in an upper surface. This housing unit 31 is made of metal such as stainless steel or a resin. In an internal space of the housing unit 31, a circular plate-shaped partition plate 36 is provided which is configured to divide the internal space vertically in a liquid-tight state.

The ultrasonic vibrator 32 is an element configured to generate ultrasonic vibration and is attached to the partition plate 36 in a state being housed in an interior of a housing body 37.

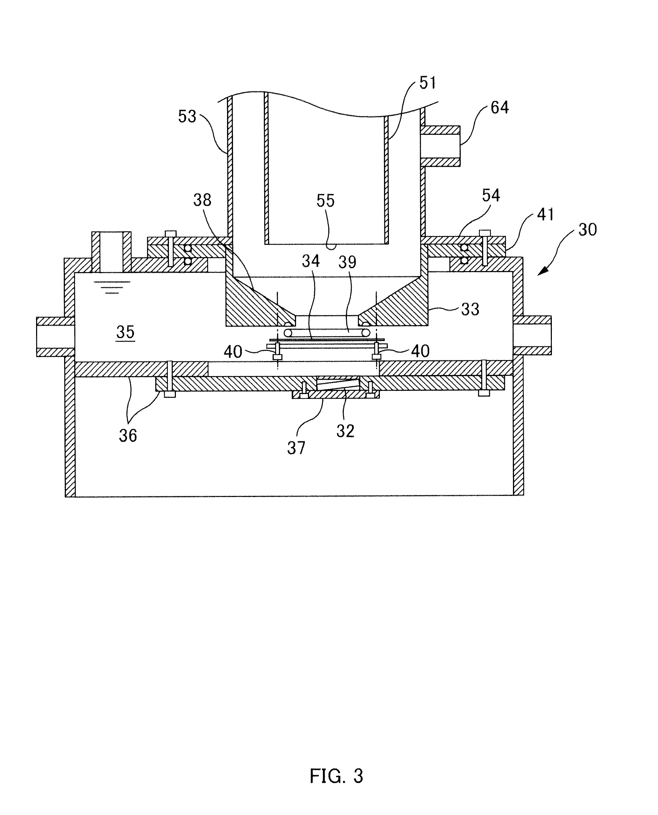

The storage portion 33 is a container configured to store hydrogen peroxide solution and includes a concave portion 38 that is concave in an inverted truncated conical shape, as illustrated in Fig. 3. In this concave portion 38, the hydrogen peroxide solution supplied from the pump 19 is stored. A bottom surface of the concave portion 38 is partitioned by the vibration plate 34. This vibration plate 34 is configured with a stainless thin plate having a thickness of approximately 0.02 mm or a resin thin plate having a thickness of approximately 0.2 mm. This vibration plate 34 is fixed to the bottom surface of the storage portion 33 from an underside using a fixing screw 40 in a state sandwiching an O-ring 39. An upper end portion 41 of the storage portion 33 is formed in a flange shape so as to be able to be connected to a lower end portion 54 of an external cylindrical portion 53 in an air-tight state.

The ultrasonic transmitting liquid 35 is stored in a space above the partition plate 36 in the housing unit 31. In an embodiment of the present invention, water is used as the ultrasonic transmitting liquid 35. The ultrasonic vibration generated by an ultrasonic vibrator 32 is transmitted to the vibration plate 34 through this ultrasonic transmitting liquid 35. The ultrasonic transmitting liquid 35 is not limited to water but any liquid can be used as long as it can transmit the ultrasonic vibration to the vibration plate 34.

By operating the ultrasonic vibrator 32 in a state where the hydrogen peroxide solution is stored in the storage portion 33, the ultrasonic vibration is transmitted to the vibration plate 34 through the ultrasonic transmitting liquid 35, and the ultrasonic vibration is transmitted through the vibration plate 34. By means of this ultrasonic vibration, the hydrogen peroxide solution in the storage portion 33 is atomized.

Subsequently, the gasifying unit 50 will be described. As illustrated in Fig. 2, the gasifying unit 50 includes an internal cylindrical portion 51, a heater 52, and an external cylindrical portion 53 and the like and is provided above the atomizing unit 30.

The internal cylindrical portion 51 is configured with a cylindrical member made of metal. The internal cylindrical portion 51 according to an embodiment of the present invention includes a stainless-portion 51A made of a stainless member and an aluminum portion 51B connected to a lower side of the stainless-portion 51 and made of an aluminum member thinner than the stainless-portion 51A. The internal cylindrical portion 51 is set at a diameter smaller than that of an opening of the concave portion 38 included in the storage portion 33. The internal cylindrical portion 51 is attached in such a state as to be directed in a vertical direction. In an attached state, the internal cylindrical portion 51 is located such that a lower end portion 55 thereof is at a height immediately above the concave portion 38 in the storage portion 33 and its shaft center is concentric with the concave portion 38.

The heater 52 is a member heated by energization and has a columnar heat generating body 56 and a heat transfer fin 57 attached in the periphery of the heat generating body 56. That is, heat from the heat generating body 56 is diffused by the fin 57. This heater 52 is arranged in an internal space of the internal cylindrical portion 51. For example, it is arranged in a range of approximately 3/4 of a length of the internal cylindrical portion 51 from the upper end side.

A flow path regulating plate 58 (internal fin) is attached between the heat generating body 56 of the heater 52 and an internal wall surface of the internal cylindrical portion 51. The flow path regulating plate 58 is configured with a metal plate in a substantially semi-circular shape with a notch portion in a semi-circular shape contacted with the heat generating body 56. This flow path regulating plate 58 is attached in a substantially horizontal direction in such a state as to cover one half of the internal space of the internal cylindrical portion 51. A plurality of the flow path regulating plates 58 are arranged in a staggered configuration at predetermined intervals in the vertical direction. For example, the flow path regulating plates 58 are arranged at certain intervals or arranged densely on the upper side and sparsely on the lower side. Moreover, they may be arranged so as to be dense in the portion corresponding to the heater 52 and sparse on the side lower than that. The heater 52 is positioned in the internal space of the internal cylindrical portion 51 by these flow path regulating plates 58. Moreover, a meandering flow path through which the gas passes is defined in the internal space of the internal cylindrical portion 51 by the flow path regulating plates 58, and the heat generated by the heat generating body 56 is transmitted to the internal cylindrical portion 51 through the flow path regulating plates 58.

A piping 59 is attached to the side surface of an upper part of the internal cylindrical portion 51. The hydrogen peroxide gas is discharged through this piping 59. A temperature of the gas flowing through the piping 59 is detected by a temperature sensor (not shown) and a detection signal is outputted to the control unit 5. An upper end portion 60 of the internal cylindrical portion 51 is formed in a flange shape and is sealed in an air-tight state by attaching an internal lid member 61.

The external cylindrical portion 53 is configured with a cylindrical member made of metal having a diameter greater than the internal cylindrical portion 51. The external cylindrical portion 53 according to an embodiment an embodiment of the present invention is configured with a stainless steel pipe having an internal diameter thereof substantially the same as the internal diameter of the concave portion 38.

The internal cylindrical portion 51 is arranged in the internal space of the external cylindrical portion 53. That is, a double pipe is configured with the external cylindrical portion 53 and the internal cylindrical portion 51. The lower end portion 54 of the external cylindrical portion 53 is connected to the upper end portion of the storage portion 33 in the air-tight state, as described above. The internal cylindrical portion 51 is covered by this external cylindrical portion 53 in a range approximately 4/5 of its length from the lower end. A piping 62 is attached on the side face of the upper part of the external cylindrical portion 53. This piping 62 is configured to introduce the carrier gas. That is, the carrier gas flows into the space (gas flow path 63 for the carrier gas) between the external cylindrical portion 53 and the internal cylindrical portion 51 through this piping 62. In an embodiment of the present invention, clear air is used as the carrier gas, and the air is introduced into the piping 62 by means of rotation of an air supply fan (carrier gas supply fan).

An upper end portion 65 of the external cylindrical portion 53 is formed in a flange shape and sealed in the air-tight state by attaching a ring-shaped external lid member 66. Moreover, a tube opening 64 through which a drain tube, allowing the hydrogen peroxide to flow therethrough, passes is provided in the side face of the lower part of the external cylindrical portion 53. A space between the drain tube and the tube opening 64 is blocked by a bushing. Thus, the tube opening 64 is sealed in the air-tight state.

### <Generation of sterilizing gas>

Subsequently, generation of a sterilizing gas by the sterilization gas generator 20 will be described. As described above, the operation of the sterilization gas generator 20 is controlled by a control signal from the control unit 5.

First, energization of the heater 52 (heat generating body 56) and rotation of the air supply fan are started. AS a result, heat generation of the heat generating body 56 and supply of the carrier gas (air) are started. Further, the pump 19 is driven to supply the hydrogen peroxide solution of the sterilizing substance cartridge 18 in a specified amount into the storage portion 33.

The carrier gas flowing through the piping 62 flows into the gas flow path 63 from the upper part of the external cylindrical portion 53, and flows downward through this gas flow path 63. That is, the carrier gas flows along the external peripheral surface of the internal cylindrical unit 51. The heat from the heat generating body 56 is transmitted to the internal cylindrical portion 51 through the flow path regulating plate 58, the fin 57, and the air, thereby heating the internal cylindrical portion 51. Heating of the internal cylindrical portion 51 causes heat exchange with the carrier gas flowing along the external peripheral surface of the internal cylindrical portion 51, thereby increasing the temperature of the carrier gas.

The carrier gas changes its flow direction at a position of the storage portion 33. That is, the carrier gas goes around the lower end portion 55 of the internal cylindrical portion 51 and flows into the internal space of the internal cylindrical portion 51, and then, rises in this internal space of the internal cylindrical portion 51. Since the heat from the heat generating body 56 is emitted to the internal space of the internal cylindrical portion 51 through the fin 57 of the heater 52, the temperature of the carrier gas rising in this internal space is further raised. In addition, a meandering flow path is formed by a plurality of the flow path regulating plates 58 in the internal space of the internal cylindrical portion 51, thereby being able to extend a travel distance of the carrier gas and reliably raising the temperature of the carrier gas.

If the temperature of the carrier gas discharged from the internal cylindrical portion 51 reaches an evaporation temperature of hydrogen peroxide, driving of the ultrasonic vibrator 32 is started. As described above, the ultrasonic vibration generated by the ultrasonic vibrator 32 is transmitted to the vibration plate 34 through the ultrasonic transmitting liquid 35. Then, the hydrogen peroxide of the storage portion 33 is atomized by the ultrasonic vibration of the vibration plate 34.

The atomized hydrogen peroxide rises through the internal space of the internal cylindrical portion 51 with the flow of the carrier gas. At this time, the carrier gas is heated while flowing through the gas flow path 63 and the internal space forms the meandering flow path, thereby being able to sufficiently heat the atomized hydrogen peroxide and reliably gasified. Moreover, since the carrier gas is heated in advance, the atomized hydrogen peroxide is prevented from adhering to the lower end portion 55 of the internal cylindrical portion 51 and being liquefied.

Furthermore, in an embodiment of the present invention, the internal cylindrical portion 51 is configured with aluminum with favorable thermal conductivity, in this respect as well, thereby being able to efficiently heat the carrier gas and reliably gasifying the hydrogen peroxide. In addition, since the carrier gas is heated in advance, heat exchange occurs between the hydrogen peroxide solution stored in the storage portion 33 and the carrier gas as well, thereby promoting evaporation of the hydrogen peroxide solution.

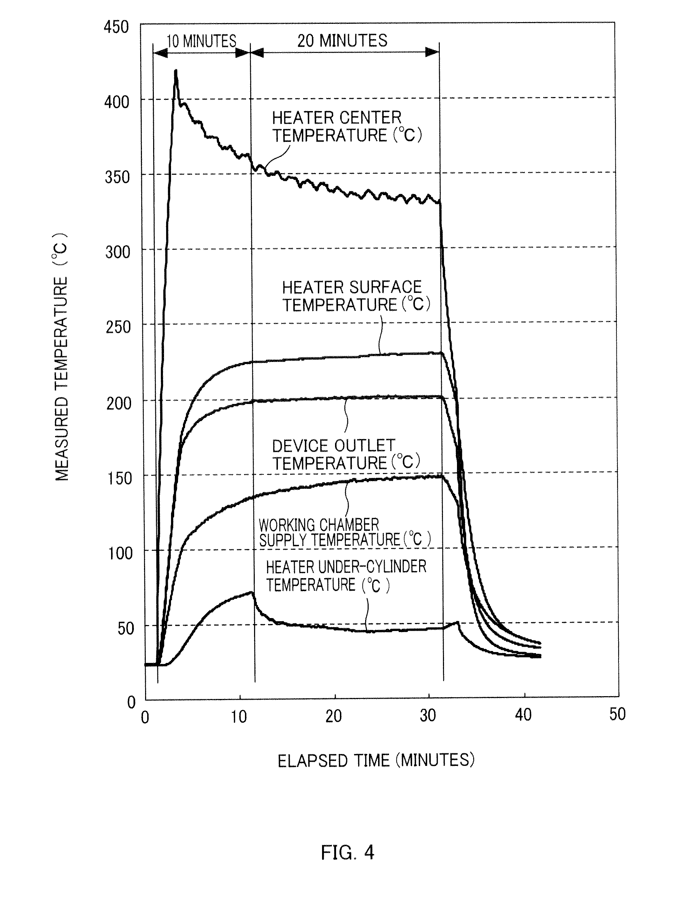

The above description can be also understood from change over time in temperature illustrated in Fig. 4. This figure is a diagram for describing change over time in heater center temperature, heater surface temperature, device outlet temperature, working chamber supply temperature, and heater under-cylinder temperature. Here, the heater center temperature is a center temperature of the heat generating body 56, and the heater surface temperature is a surface temperature of the heat generating body 56. The device outlet temperature is a gas temperature at the outlet of the internal cylindrical portion 51, and the working chamber supply temperature is a temperature of the gas supplied to the working chamber 1. The heater under-cylinder temperature is a gas temperature in the vicinity of the lower end portion 55 of the internal cylindrical portion 51.

In this example, atomization of hydrogen peroxide by the ultrasonic vibrator 32 is started 10 minutes after the start of energization of the heater 52. Then, the gasification of a predetermined amount of the hydrogen peroxide solution is completed in 20 minutes.

In this example, the heater center temperature is raised to approximately 425°C, and thereafter lowered to approximately 340°C. The heater surface temperature is approximately 225°C at the start of atomization of the hydrogen peroxide, and this temperature is maintained thereafter. The device outlet temperature is approximately 200°C at the start of atomization of the hydrogen peroxide, and this temperature is maintained thereafter. The working chamber supply temperature is approximately 140°C at the start of atomization of the hydrogen peroxide, and then is raised by approximately 10°C in 20 minutes thereafter. The heater under-cylinder temperature is approximately 70°C at the start of atomization, but is lowered to slightly below 50°C by atomization of the hydrogen peroxide, and this temperature is maintained thereafter.

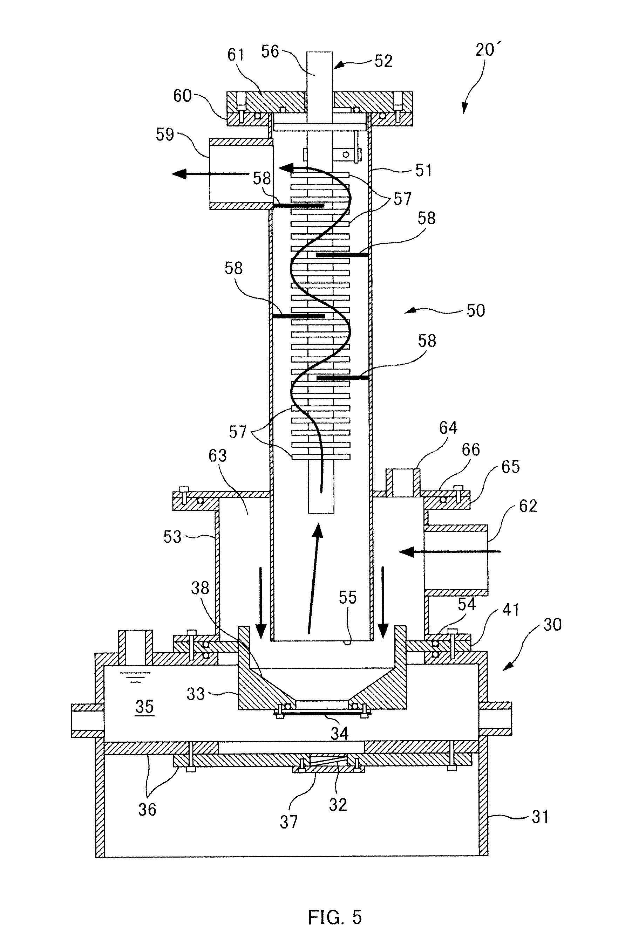

Here, a sterilization gas generator 20ʹ of a reference example will be described. In the sterilization gas generator 20' of the reference example illustrated in Fig. 5, the same reference numerals are given to the portions corresponding to those in the sterilization gas generator 20 described in Figs. 2 and 3, and the descriptions thereof will be omitted. A difference between the sterilization gas generator 20' in the reference example and the sterilization gas generator 20 in Fig. 2 is firstly a length of the external cylindrical portion 53. That is, in this reference example, the length of the external cylindrical portion 53 is a length covering approximately 1/4 of the length of the internal cylindrical portion 51 from the lower end side of the internal cylindrical portion 51. In other words, the internal cylindrical portion 51 has a portion covered, which is not heated by the heater 52. Further, it is also different from the sterilization gas generator 20 in Fig. 2 that the internal cylindrical portion 51 is made of stainless steel.

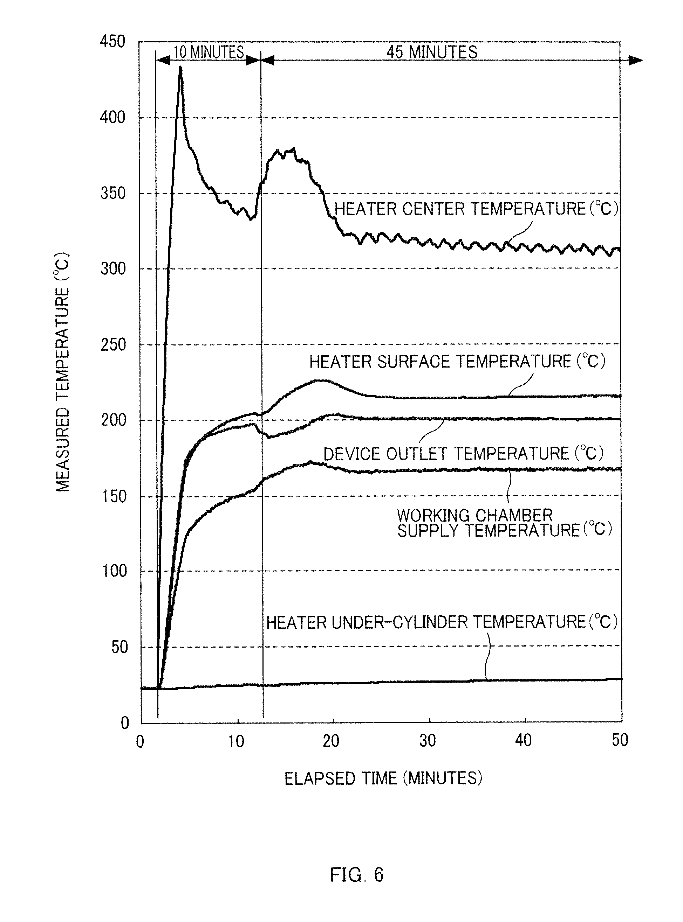

In the reference example, as illustrated in Fig. 6, the heater 52 is controlled so that the device outlet temperature becomes 200°C which is the same as that in an embodiment of the present invention. As is known from comparison with Fig. 4, the heater 52 is heated again at the start of atomization, causing unstable control. This difference is considered to be caused more greatly by a difference in the heater under-cylinder temperature. That is, the heater under-cylinder temperature in the reference example is substantially constant at approximately 25°C, and is lower by approximately 20°C than that in an embodiment of the present invention. It is considered that gasification required longer time in the reference example due to the difference in the heater under-cylinder temperature.

### <Conclusion>

The sterilization gas generator 20 according to an embodiment of the present invention includes: the atomizing unit 30 configured to atomize the hydrogen peroxide stored in the storage portion 33 by applying ultrasonic vibration; the heater 52 provided above this atomizing unit 30 and configured to heat and gasify the hydrogen peroxide atomized in the atomizing unit 30; the internal cylindrical portion 51, made of metal, that has the internal space in which this heater 52 is arranged and that is configured to guide the hydrogen peroxide upward which has been atomized in the atomizing unit 30 and flows with the carrier gas; and the external cylindrical portion 53 having the internal space in which the internal cylindrical portion 51 is arranged such that a double pipe is configured, the external cylindrical portion configured to form the gas flow path 63 for the carrier gas flowing downward the storage portion 33 between itself and the internal cylindrical portion 51. Then, the configuration is such that the carrier gas flowing through the gas flow path 63 is brought into contact with the internal cylindrical portion 51 heated by the heater 52, so that the heated carrier gas is introduced into the storage portion 33. As a result, the hydrogen peroxide solution in the storage portion 33 is evaporated also through heat exchange with the carrier gas, thereby being able to reduce the amount of the hydrogen peroxide solution remaining in the storage portion 33 to the smallest amount possible.

Moreover, the flow path regulating plate 58 made of a metal plate, which regulates a flow direction of the atomized hydrogen peroxide and has thermal conductivity, is attached between the internal wall surface of the internal cylindrical portion 51 and the heater 52, thereby being able to sufficiently heat and reliably gasify the atomized hydrogen peroxide.

Moreover, the external cylindrical portion 53 is made of stainless steel having thermal conductivity lower than that of the internal cylindrical portion 51 made of aluminum, thereby being able to suppress the escape of the heat to the exterior and sufficiently heat the carrier gas flowing through the gas flow path 63.

The first embodiment has been described hereinabove, but the above description is given for facilitating understanding of the present invention and is not intended to limit the present invention. It is needless to say that the present invention is capable of changes or improvements without departing from the gist thereof and also includes their equivalents. For example, configurations may be made according to the present invention as each of embodiments described below.

### <<Second embodiment>>

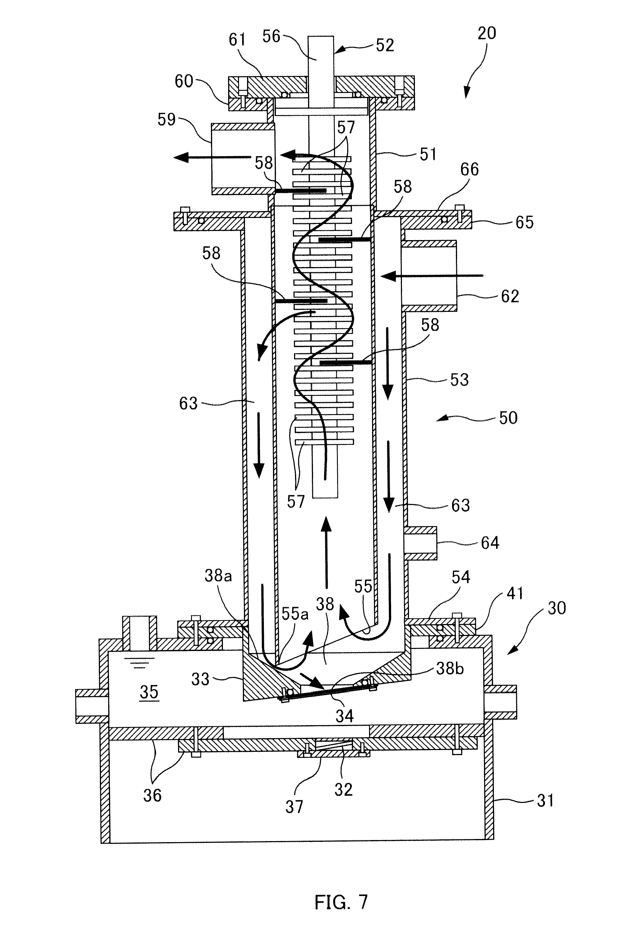

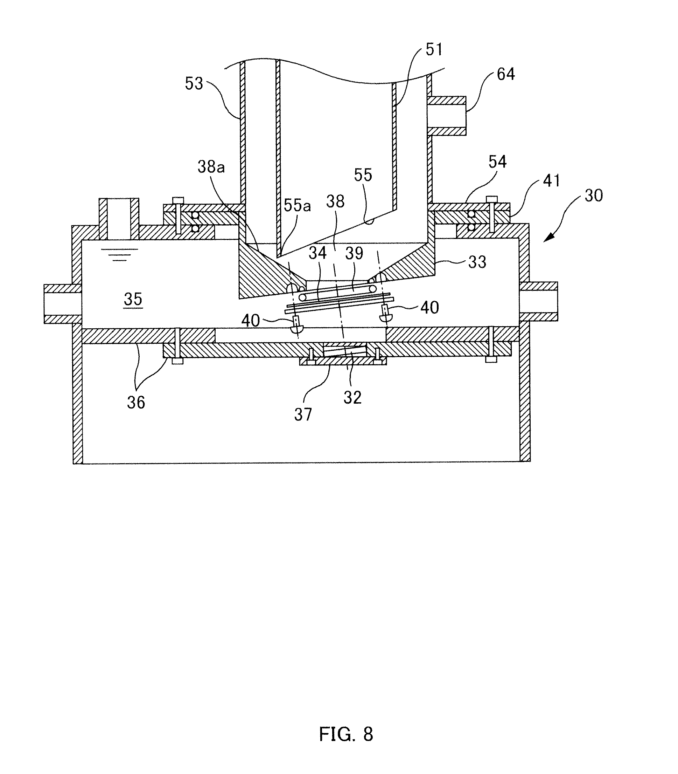

In a second embodiment of the present invention, the lower end portion 55 of the internal cylindrical portion 51 is in a shape different from that in a first embodiment of the present invention. For example, as illustrated in Figs. 7 and 8, the internal cylindrical portion 51 according to a second embodiment of the present invention is configured with a cylindrical member obtained by cutting the lower end portion 55 thereof diagonally with respect to the longitudinal direction of the cylinder.

The internal cylindrical portion 51 is positioned so that the shaft center thereof becomes concentric with the concave portion 38 in the horizontal direction. Moreover, the internal cylindrical portion 51 is positioned at a height where a top portion 55a of the lower end portion 55 is close to an inclined surface 38a of the concave portion 38. Here, the height close to the inclined surface 38a of the concave portion 38 indicates such a height that a narrow space is formed between the top portion 55a and the inclined surface 38a, that is, such a height that a flow velocity of the carrier gas passing through this space is increased as compared with the carrier gas passing through other portions.

As such, in a second embodiment of the present invention, the lower end portion 55 of the internal cylindrical portion 51 is cut diagonally with respect to the longitudinal direction, and the top portion 55a of this lower end portion 55 is arranged at the height close to the inclined surface 38a of the concave portion 38. Thus, in the lower end portion 55 of the internal cylindrical portion 51, a difference can be made in the flow velocity of the carrier gas between the top portion 55a side and the opposite side. That is, the flow velocity of the carrier gas flowing through the top portion 55a side can be increased as compared with the flow velocity of the carrier gas flowing through the opposite side. As a result, the carrier gas whose flow velocity is increased can be blown on the hydrogen peroxide solution stored in the concave portion 38, which assists movement of the atomized hydrogen peroxide and can reliably gasify the hydrogen peroxide.

Moreover, in an embodiment of the present invention, the bottom surface 38b (vibration plate 34) of the concave portion 38 is provided in such a state as to be inclined downward toward the top portion 55a side of the lower end portion 55. Thus, when the amount of the hydrogen peroxide solution stored in the concave portion 38 decreases, the hydrogen peroxide solution is collected on the top portion side of the lower end portion due to this downward inclination. As a result, the movement of the atomized hydrogen peroxide is also assisted, thereby being able to reliably gasify the hydrogen peroxide.

### <<Third embodiment>>

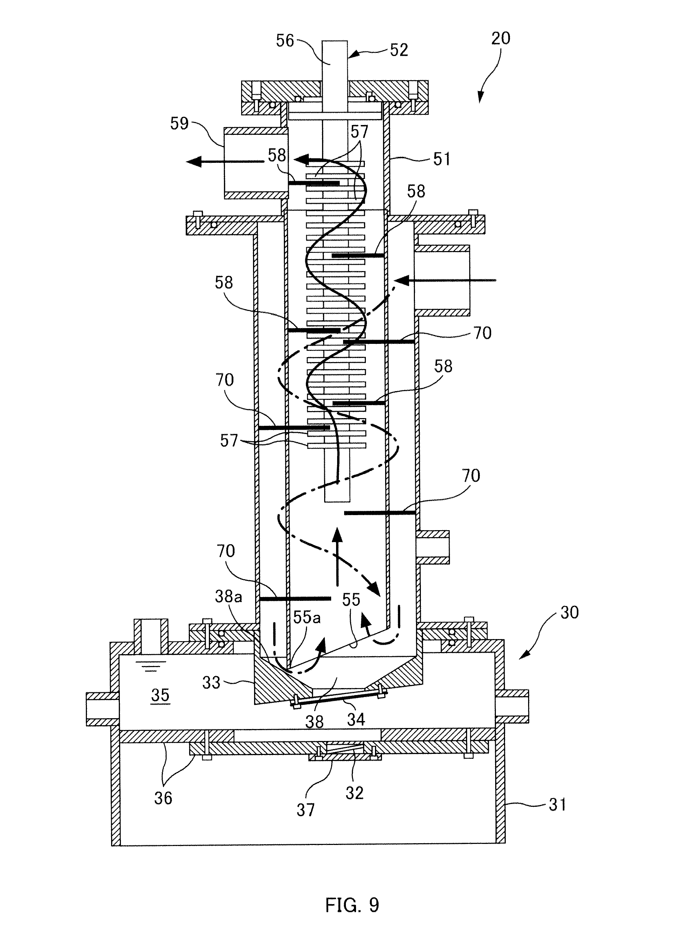

In a third embodiment of the present invention, as illustrated in Fig. 9, an external flow path regulating plate 70 (external fin) made of a metal plate, which regulates a flow direction of the carrier gas as well as has thermal conductivity, is attached between an external wall surface of the internal cylindrical portion 51 and an internal wall surface of the external cylindrical portion 53, in addition to the configuration of a second embodiment of the present invention.

This external flow path regulating plate 70 is configured with a band-shaped metal plate curved in a semicircular shape. Specifically, the external flow path regulating plate 70 is configured with a plate material in a shape obtained by splitting a ring-shaped stainless plate, having a width according to an interval between the internal cylindrical portion 51 and the external cylindrical portion 53, into halves along a line passing the center of the ring.

The external flow path regulating plate 70 is attached substantially in the horizontal direction between the external wall surface of the internal peripheral unit 51 and the internal wall surface of the external cylindrical portion 53, in such a state as to cover one half of the internal cylindrical portion 51 and the external cylindrical portion 53. Moreover, as to the external flow path regulating plate 70, a plurality of plates are arranged in a staggered configuration at predetermined intervals in the vertical direction. That is, similarly to the flow path regulating plate 58, they may be arranged at the constant intervals so as to be dense on the upper side and to be sparse on the lower side or arranged so as to be dense in the portion corresponding to the heater 52 and sparse on the side lower than that. These external flow path regulating plates 70 define the gas flow path 63 for the carrier gas so as to meander. As indicated by a one-dot chain line arrow in Fig. 9, the carrier gas flows downward while meandering through this gas flow path 63.

Therefore, in an embodiment of the present invention, a time period during which the carrier gas is being in contact with the internal cylindrical portion 51 can be made longer than that in the above-described embodiment of the present invention. As a result, the sufficiently heated carrier gas can be allowed to flow to the storage portion 33. AS a result, movement of the atomized hydrogen peroxide is assisted, and the hydrogen peroxide can be reliably gasified.

### <<Fourth embodiment>>

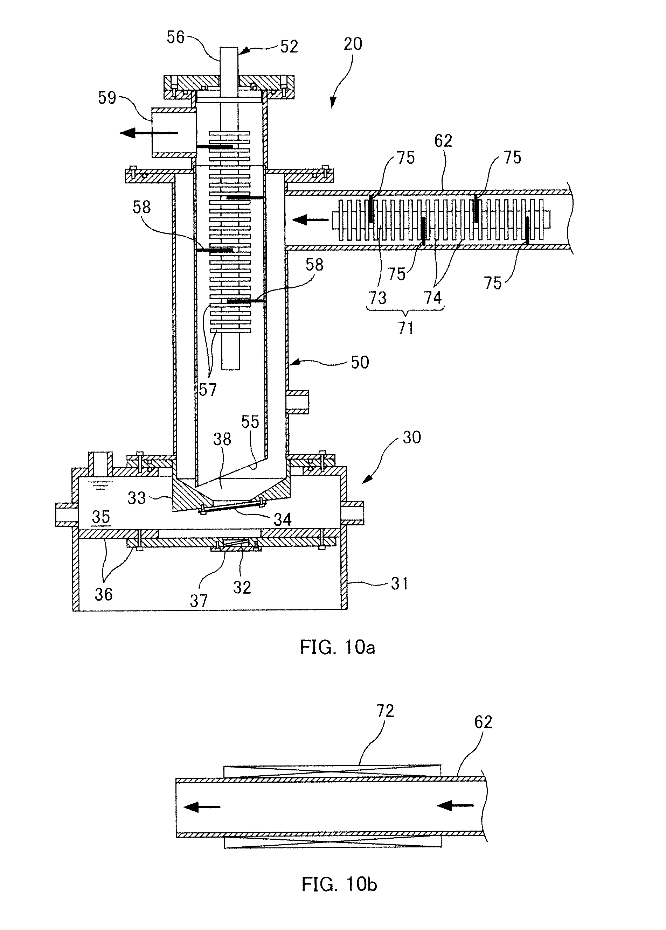

A fourth embodiment of the present invention has characteristics in that preheating heaters 71 and 72 configured to preheat the carrier gas are provided at some midpoint in the piping 62 (gas introduction pipe) configured to introduce the carrier gas, in addition to the configuration of a second embodiment as illustrated in Fig. 10.

The preheating heater may be the preheating heater 71 including a columnar heat generating body 73 and a heat transfer fin 74 attached in the periphery of the heat generating body 73 as illustrated in Fig. 10(a), or may be the preheating heater 72 in a cylindrical shape attached in such a state as to surround the external peripheral surface of the piping 62 as illustrated in Fig. 10(b).

The preheating heater 71 in Fig. 10 (a) is attached to the piping 62 through a flow path regulating plate 75. This flow path regulating plate 75 is configured, similarly to the flow path regulating plate 58 according to a first embodiment of the present invention. That is, the flow path regulating plate 75 is configured with a metal plate in a substantially semicircular shape with a notch portion in a semicircular shape contacted with the heat generating body 73. As to this flow path regulating plate 75 as well, a plurality of plates are arranged in a staggered configuration in such a state as to cover one half of the internal space of the cylindrical piping 62. These flow path regulating plates 75 define a meandering flow path through which the gas passes in the internal space of the piping 62, thereby being able to sufficiently transmit the heat from the preheating heater 71 to the carrier gas.

In an embodiment of the present invention, the carrier gas preheated by the preheating heaters 71 and 72 is heated by being brought into contact with the internal cylindrical portion 51 in the gas flow path 63. Thus, the carrier gas can be sufficiently raised in temperature, thereby being able to further reliably gasify the atomized hydrogen peroxide.

### <<Example 2>>

Subsequently, an example 2 of the present invention will be described.

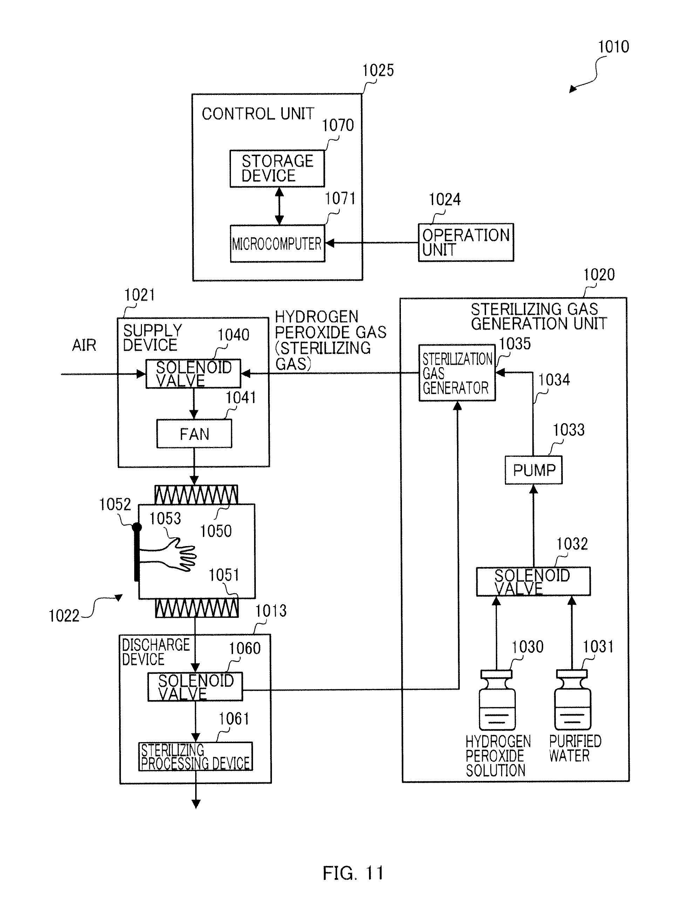

Fig. 11 is a diagram illustrating a configuration of an isolator 1010 according to an embodiment of the present invention. The isolator 1010 is a device configured to conduct a work on a cell and the like in a sterilized environment, and includes a sterilizing gas generation unit 1020, a supply device 1021, a working chamber 1022, a discharge device 1023, an operation unit 1024, and a control unit 1025.

The sterilizing gas generation unit 1020 is an apparatus unit configured to generate a sterilizing gas, and includes tanks 1030 and 1031, a solenoid valve 1032, a pump 1033, a pipe 1034, and a sterilization gas generator 1035. Operations of the solenoid valve 1032, the pump 1033, and the sterilization gas generator 1035 are controlled by the control unit 1025.

The tank 1030 is configured to store a hydrogen peroxide solution (aqueous solution in which hydrogen peroxide (H₂O₂) is dissolved) and the tank 1031 is configured to store purified water.

The solenoid valve 1032 is a solenoid valve configured to connect the tank 1030 or the tank 1031 to the pump 1033 under control from the control unit 1025.

The pump 1033 pumps up the hydrogen peroxide solution from the tank 1030 when the solenoid valve 1032 selects the tank 1030, and supplies it to the sterilization gas generator 1035 through the pipe 1034. On the other hand, the pump 1033 pumps up the purified water from the tank 1031 when the solenoid valve 1032 selects the tank 1031, and supplies it to the sterilization gas generator 1035 through the pipe 1034.

The sterilization gas generator 1035 is configured to generate a hydrogen peroxide gas which is a sterilizing gas on the basis of the hydrogen peroxide solution supplied from the pump 1033, and supplies it together with air which is the carrier gas to the supply device 1021. The details of the sterilization gas generator 1035 will be described later.

The supply device 1021 is a device configured to supply the supplied hydrogen peroxide gas or the air in the exterior of the isolator 1010 to the working chamber 1022, and includes a solenoid valve 1040 and a fan 1041.

The solenoid valve 1040 is configured to supply the hydrogen peroxide gas or the external air to the fan 1041 under control of the control unit 1025. The fan 1041 is configured to supply the hydrogen peroxide gas supplied from the solenoid valve 1040 or the air to the working chamber 1022.

The working chamber 1022 is a space in which a work on a cell is conducted, and air filters 1050 and 1051, a door 1052, and a working glove 1053 are provided in the working chamber 1022.

The air filter 1050 is a filter configured to remove dusts and the like contained in the hydrogen peroxide gas or the air supplied from the fan 1041. The air filter 1051 is a filter configured to remove dusts and the like contained in a gas and the like to be discharged from the working chamber 1022. An HEPA (High Efficiency Particulate Air) filter, for example, is used as the air filters 1050 and 1051.

The door 1052 is provided on the front surface of the working chamber 1022 in such a manner as to be capable of being opened/closed, in order to convey a cell and the like to the working chamber 1022.

The working glove 1053 is attached to an opening portion (not shown) provided in the door 1052 so that a worker can work on a cell and the like in the working chamber 1022 in a state where the door 1052 is closed. In a state where the door 1052 is closed, the working chamber 1022 is sealed.

The discharge device 1023 is a device configured to discharge the hydrogen peroxide gas or a gas such as air from the working chamber 1022, and includes a solenoid valve 1060 and a sterilization processing device 1061.

The solenoid valve 1060 is configured to supply a gas outputted from the air filter 1051 to either of the sterilization processing device 1061 or the sterilization gas generator 1035 under control from the control unit 1025. If the output from the solenoid valve 1060 is supplied to the sterilization gas generator 1035, the gas in the working chamber 1022 is circulated.

The sterilization processing device 1061 is provided with a catalyst, and is configured to render the gas outputted from the solenoid valve 1060 harmless and apply sterilization processing thereto, then output it to the exterior of the isolator 1010.

The operation unit 1024 is an operation panel and the like using which a user set an operation of the isolator 1010. An operation result of the operation unit 1024 is transmitted to the control unit 1025, and the control unit 1025 is configured to control each of the blocks of the isolator 1010 on the basis of the operation result.

The control unit 1025 is a device configured to supervise and control the isolator 1010, and includes a storage device 1070 and a microcomputer 1071.

The storage device 1070 is configured to store program data to be executed by the microcomputer 1071 and various data. The microcomputer 1071 is configured to realize various functions by executing the program data stored in the storage device 1070. For example, when an instruction to generate a sterilizing gas is outputted from the operation unit 1024, the microcomputer 1071 executes a predetermined program for generating the sterilizing gas and controls the pump 1033 and the like.

The sterilizing gas generation unit 1020 and the control unit 1025 are equivalent to a sterilization substance generator. Moreover, the tank 1030, the solenoid valve 1032, the pump 1033, and the pipe 1034 are equivalent to a first supply unit, and the tank 1031, the solenoid valve 1032, the pump 1033, and the pipe 1034 correspond to a second supply unit.

### == Details of sterilization gas generator 1035 ==

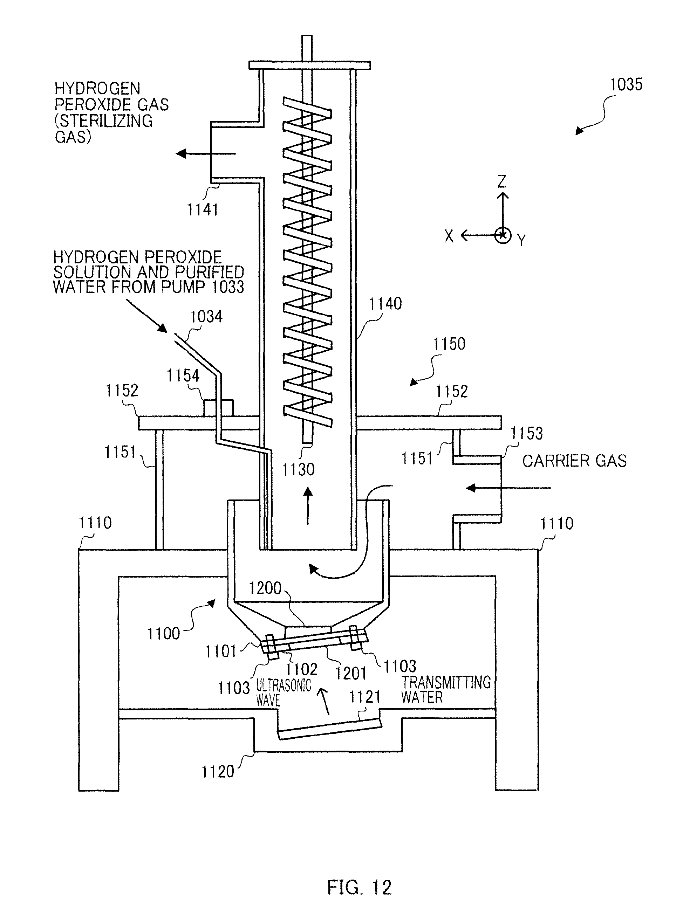

Fig. 12 is a side view of the sterilization gas generator 1035. In Fig. 12, a part of blocks is illustrated in a sectional view. The sterilization gas generator 1035 includes a cup 1100 configured to store the hydrogen peroxide solution and a holding base 1110 configured to hold the cup 1100. An opening portion is provided on an upper side (+Z direction) and a lower side (-Z direction) in the cup 1100. A vibration plate 1101 is fixed to the opening portion 200 on the lower side of the cup 1100 so as to close the opening portion 200, using a ring-shaped attaching plate 102 including an opening portion 201 and a bolt 1103. Thus, the vibration plate 1101 is configured to vibrate upon supply of the ultrasonic waves and atomize the hydrogen peroxide solution stored in the cup 1100. The cup 1100, the vibration plate 1101, the attaching plate 1102, and the bolt 1103 are equivalent to a storage portion.

The interior of the holding base 1110 configured to hold the cup 1100, a partition plate 1120 is attached so as to store the transmitting water for vibrating the vibrating plate 1101 of the cup 1100. Moreover, the partition plate 1120 is provided with an ultrasonic vibrator 1121 configured to vibrate the vibrating plate 1101 with the ultrasonic waves. The transmitting water stored inside the holding base 1110 can be replaced through a port (not shown) provided in the side face of the holding base 1110. The cup 1100, the vibrating plate 1101, the attaching plate 1102, the bolt 1103, and the ultrasonic vibrator 1121 are equivalent to an atomizing unit.

On the upper side of the holding base 1110, a supply pipe 1140 configured to supply the hydrogen peroxide gas to the exterior and a support member 150 configured to support the supply pipe 1140 are provided. The support member 150 includes a cylindrical member 1151 mounted on the upper surface of the holding base 1110 and a flange 1152 provided on the upper surface of the cylindrical member 1151.

The cylindrical member 1151 has a diameter greater than the diameter of the cylindrical supply pipe 1140, and a port 1153 supplied with a carrier gas (air in this case) is provided in the side face on the -X side of the cylindrical member 1151. The flange 1152 is configured to close the opening portion on the upper surface of the cylindrical member 1151 and allow the supply pipe 1140 to pass therethrough at the center thereof. Moreover, the upper surface of the flange 1152 is provided with a port 1154 that is configured to allow the pipe 1034, through which the hydrogen peroxide solution and the purified water is supplied, to pass therethrough. The pipe 1034 is fixed to the side face of the supply pipe 1140 so that the hydrogen peroxide solution and the like can be supplied to the cup 1100 through the port 1154 and the opening portion provided in the side face of the supply pipe 1140.

Moreover, above the cup 1100, a heater 1130 configured to heat and gasify the atomized hydrogen peroxide solution is provided. The hydrogen peroxide gas heated and gasified by the heater 1130 is outputted from a port 1141 provided in the supply pipe 1140 together with the supplied carrier gas. The port 1141 is connected to the solenoid valve 1040 of the above-described supply device 1021 through a pipe. As such, the hydrogen peroxide solution atomized in the cup 1100 is supplied as the hydrogen peroxide gas to the supply device 1021 from the port 1141. The heater 1130 and the supply pipe 1140 are equivalent to a gasifying unit.

### == Details of microcomputer 1071 ==

A description will be given of a functional block realized by the microcomputer 1071 when the hydrogen peroxide gas is generated in the sterilizing gas generation unit 1020. It is assumed that a user operates the operation unit 1024 in advance and sets an initial amount of the hydrogen peroxide solution supplied to the sterilization gas generator 1035. Moreover, the microcomputer 1071 is configured to cause the storage device 1070 to store information about the amount of the initially supplied hydrogen peroxide solution (hereinafter referred to as a predetermined amount A1) on the basis of the operation result of the operation unit 1024. The predetermined amount A1 of the information stored in the storage device 1070 is read and used by the microcomputer 1071 as appropriate.

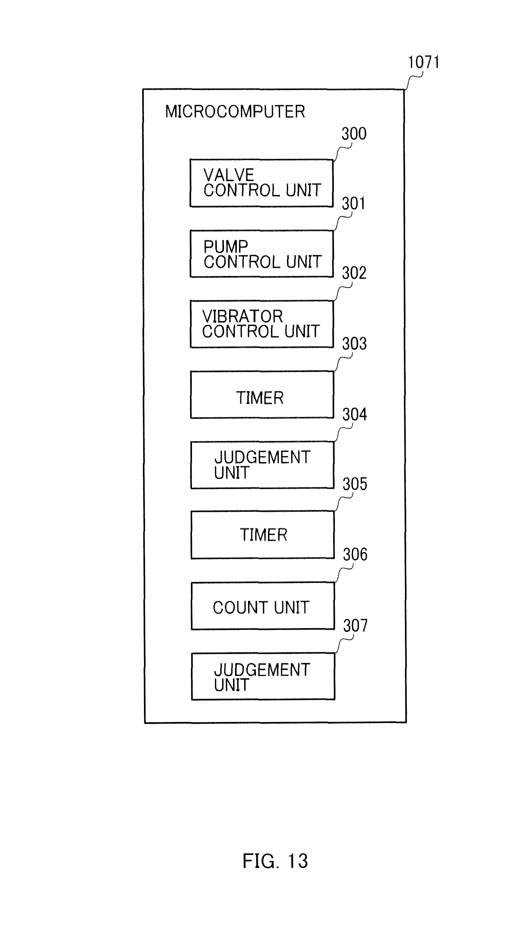

When an instruction to generate the hydrogen peroxide gas is inputted from the operation unit 1024, the microcomputer 1071 executes a predetermined program and realizes functions of a valve control unit 300, a pump control unit 301, a vibrator control unit 302, timers 303 and 305, judgment units 304 and 307, and a count unit 306 as illustrated in Fig. 13. The valve control unit 300, the pump control unit 301, and the vibrator control unit 302 are equivalent to a control unit. Moreover, the judgment units 304 and 307 are equivalent to a judgment unit, and the timers 303 and 305 and the judgment units 304 and 307 are equivalent to a determination unit.

The valve control unit 300 switches the solenoid valve 1032 to the tank 1030 side on the basis of the instruction to start processing from the operation unit 1024. Moreover, the valve control unit 300 switches the solenoid valve 1032 to the tank 1031 side on the basis of the instruction of a water feeding instruction which will be described later.

When the valve control unit 300 switches the solenoid valve 1032 to the tank 1030 side, the pump control unit 301 operates the pump 1033. Then, the pump control unit 301 controls the pump 1033 so that the predetermined amount A1 of the hydrogen peroxide solution is supplied to the sterilization gas generator 1035. Moreover, the pump control unit 301 operates the pump 1033 on the basis of the water feeding instruction. At this time, the pump control unit 301 controls the pump 35 so that a predetermined amount B1 of the purified water is supplied to the sterilization gas generator 1035. Information about the predetermined amount B1 is assumed to be stored in advance in the storage device 1070, for example.

The vibrator control unit 302 is configured to determine whether or not the supply of the predetermined amount A1 of the hydrogen peroxide solution has been completed, and when the supply of the hydrogen peroxide solution is completed, it operates the ultrasonic vibrator 1121 so as to atomize the hydrogen peroxide solution.

The timer 303 (first timer) starts measuring time, when the supply of the predetermined amount A1 of the hydrogen peroxide solution to the sterilization gas generator 1035 is completed.

When the timer 303 has measured a predetermined time TA (first time period), the judgment unit 304 judges that the amount of the hydrogen peroxide solution has reached a predetermined amount A2 (< A1).

If the hydrogen peroxide solution is continuously atomized, the amount of the hydrogen peroxide solution stored in the cup 1100 gradually decreases with gradual increase in the concentration of the remaining hydrogen peroxide solution. Moreover, since the hydrogen peroxide solution with high concentration is difficult to be gasified, the hydrogen peroxide solution with high concentration may remain in the cup 1100. In an embodiment of the present invention, for example, the amount of the hydrogen peroxide solution remaining in the end acquired when atomization is continuously executed is referred to as a predetermined amount A2 (first predetermined amount).

Moreover, when the judgment unit 304 judges that the amount of the hydrogen peroxide solution has reached the predetermined amount A2, it outputs a water feeding instruction to the valve control unit 300 and the pump control unit 301, when judging that the amount of the hydrogen peroxide solution has reached the predetermined amount A2. That is, the judgment unit 304 is configured to output the water feeding instruction to dilute the remaining hydrogen peroxide solution when it determines that the hydrogen peroxide solution is atomized and the amount of the hydrogen peroxide solution has decreased to the predetermined amount A2. The amount (a predetermined amount B1) of the purified water supplied to the sterilization gas generator 1035 is set so as to become greater than the predetermined amount A2 on the basis of the water feeding instruction.

Moreover, the above-described time TA is calculated by the judgment unit 304, for example, by using a decreased amount of the hydrogen peroxide solution per unit time experimentally obtained when the hydrogen peroxide solution is atomized and the predetermined amounts A1 and A2. The decreased amount per unit time and the predetermined amount A2 are assumed to be stored in the storage device 1070 in advance.

The timer 305 (second timer) starts measuring time when the supply of the predetermined amount B1 of the purified water to the sterilization gas generator 1035 is completed.

The count unit 306 is configured to count the number of times the purified water is supplied to the sterilization gas generator 1035. Specifically, the count unit 306 increments a count value by "1" each time the purified water is supplied.

when the count value of the count unit 306 is smaller than a predetermined value, the judgment unit 307 judges that the amount of the diluted hydrogen peroxide solution has reached a predetermined amount B2 (second predetermined amount) when the timer 305 has measured a predetermined time TB (second time period). Moreover, when the judgment unit 307 judges that the amount of the diluted hydrogen peroxide solution has reached the predetermined amount B2, it outputs the water feeding instruction. Moreover, when the count value of the count unit 306 has reached a predetermined count value, the judgment unit 307 controls the vibrator control unit 302 so that the atomization is stopped after an elapse of predetermined time. The time TB is calculated by the judgment unit 307, for example, by using a decreased amount of the hydrogen peroxide solution per unit time experimentally obtained when the hydrogen peroxide solution is atomized and the predetermined amounts B1 and B2, Moreover, the predetermined amount B2 is assumed to be stored in the storage device 1070 in advance.

### == Example of processing of microcomputer 1071 ==

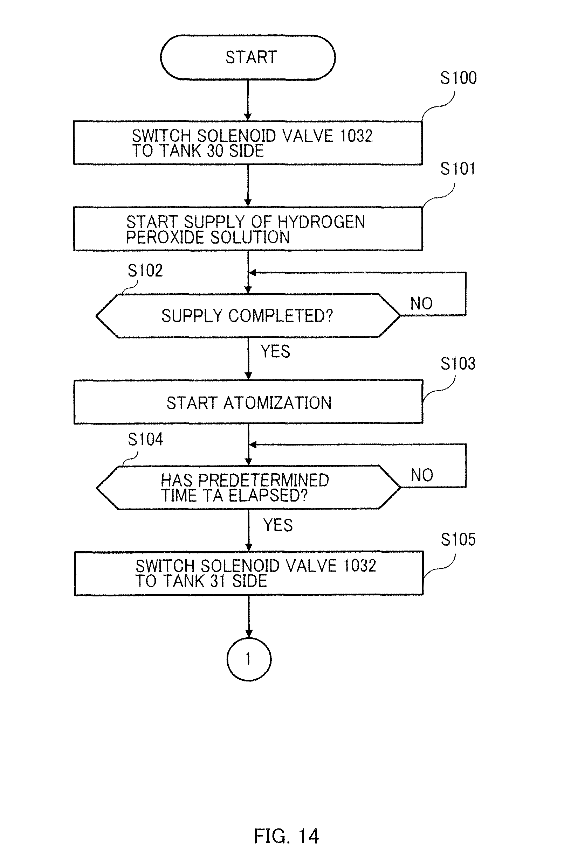

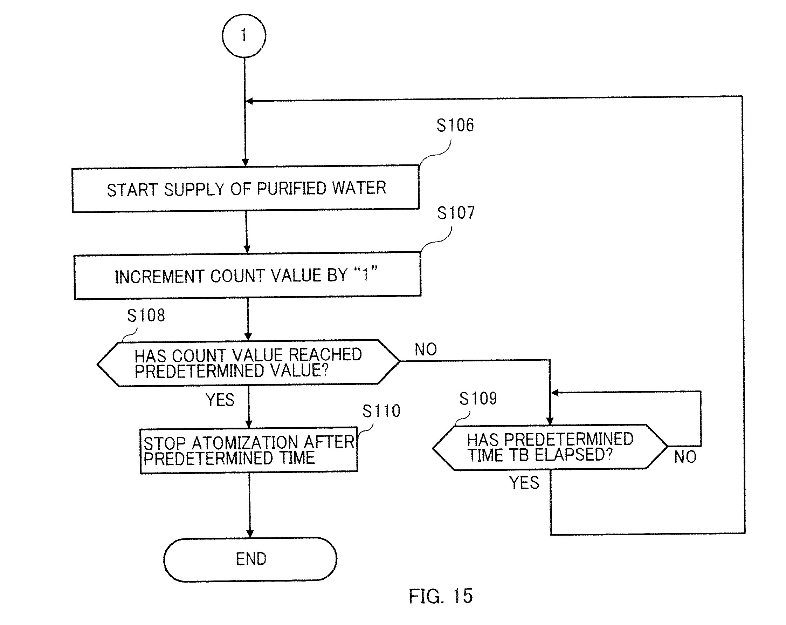

A description will be given, by referring to Figs. 14 and 15, of an example where the hydrogen peroxide gas is generated in the sterilizing gas generation unit 1020 using processing executed by the microcomputer 1071.

First, the valve control unit 300 switches the solenoid valve 1032 to the tank 1030 side (S100). Then, the pump control unit 301 operates the pump 1033, thereby starting supply of the predetermined amount A1 of the hydrogen peroxide solution to the sterilization gas generator 1035 (S101). The predetermined amount A1 of the hydrogen peroxide solution is supplied to the cup 1100 of the sterilization gas generator 1035 through the pipe 1034 as described above.

The vibrator control unit 302 determines whether or not the supply of the predetermined amount A1 of the hydrogen peroxide solution is completed (S102). When the supply of the hydrogen peroxide solution is completed (S102: YES), the vibrator control unit 302 operates the ultrasonic vibrator 1121 of the sterilization gas generator 1035 and starts atomization (S103). Further, when the hydrogen peroxide solution has been supplied to the sterilization gas generator 1035, the timer 303 starts measuring time. Moreover, the judgment unit 304 judges whether or not the timer 303 has measured the predetermined time TA (S104). When the timer 303 has measured the predetermined time TA (S104 : YES), the judgment unit 304 judges that the amount of the hydrogen peroxide solution has decreased to the predetermined amount A2, and outputs the water feeding instruction to the solenoid valve 1032 and the pump 1033.

The valve control unit 300 switches the solenoid valve 1032 to the tank 1031 side on the basis of the water feeding instruction (S105). Moreover, the pump control unit 301 operates the pump 1033 on the basis of the water feeding instruction, thereby starting the supply of the purified water to the sterilization gas generator 1035 (S106). Moreover, when processing S106 is executed and the purified water is supplied, the count unit 306 increments the count value by "1" (S107). Further, the timer 305 starts measuring time when the purified water has been supplied, that is, when the supply of the predetermined amount B1 of the purified water is completed.

When the processing S106 is executed and the predetermined amount B1 of the purified water is supplied to the sterilization gas generator 1035, the hydrogen peroxide solution remaining in the sterilization gas generator 1035 is diluted. At this time, the theoretical remaining amount of the hydrogen peroxide solution stored in the sterilization gas generator 1035 is the sum of the predetermined amount A2 and the predetermined amount B1. As described above, the predetermined amount B1, which is the amount of the purified water, is set to become greater than the predetermined amount A2 , which is a remaining amount of the hydrogen peroxide solution. Thus, the predetermined amount A2 of the hydrogen peroxide solution is diluted by a factor of at least two or more.

Then, the judgment unit 307 judges whether or not the count value of the count unit 306 has reached the predetermined value (S108). If the count value of the count unit 306 is not the predetermined value (S108: NO), the judgment unit 307 judges whether or not the timer 305 has measured the predetermined time TB (S109). If the timer 305 has measured the predetermined time TB (S109: YES), the judgment unit 307 judges that the amount of the diluted hydrogen peroxide solution has decreased to the predetermined amount B2, and outputs the water feeding instruction to the solenoid valve 1032 and the pump 1033, so as to start supply of the purified water (S106).

On the other hand, if the count value of the count unit 306 has reached the predetermined value (S108: YES), the judgment unit 307 controls the vibrator control unit 302 so that the atomization is stopped after an elapse of a predetermined time (S110).

As such, the sterilization gas generator 1035 can generate the hydrogen peroxide gas while diluting the remaining high concentration of hydrogen peroxide with purified water.

### == Example of operation of sterilizing gas generation unit 1020

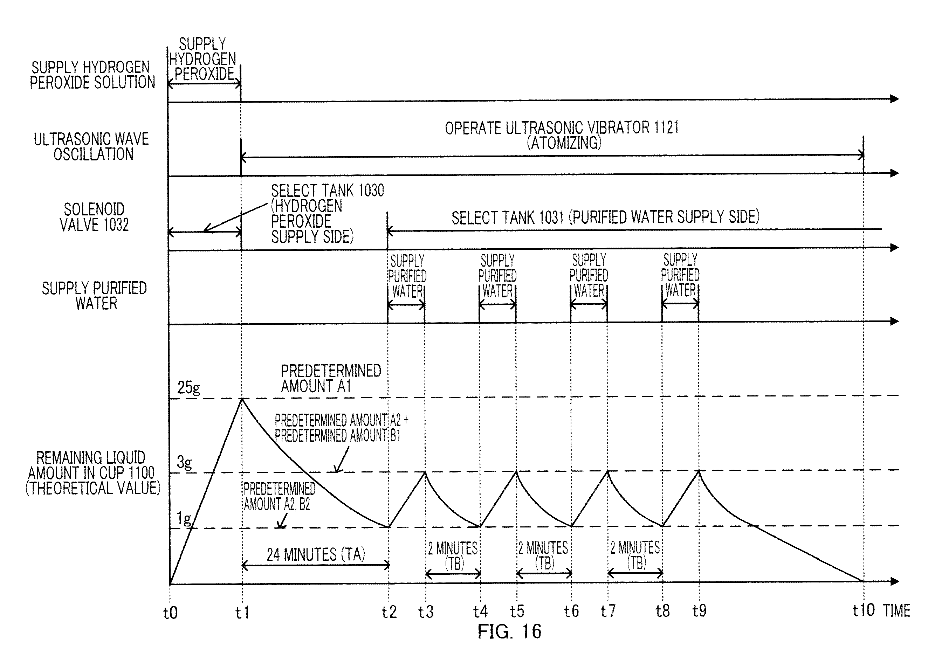

== A description will be given, by referring to Fig. 16, of an example of operations of the sterilizing gas generation unit 1020 and the control unit 1025 when the hydrogen peroxide gas is generated. Here, it is assumed that the user operates the operation unit 1024, and an instruction to generate the hydrogen peroxide gas is outputted from the operation unit 1024 to the microcomputer 1071. Furthermore, the microcomputer 1071 is assumed to execute processing as illustrated in the above-described Figs. 14 and 15 on the basis of the inputted instruction.

Here, it is assumed that the predetermined amount A1 is 25 g, the predetermined amount A2 is 1 g, the predetermined amount B1 is 2 g, and the predetermined amount B2 is 1 g, for example. Furthermore, the sterilization gas generator 1035 according to an embodiment of the present invention is assumed to atomize 1 g of the hydrogen peroxide solution per minute. Thus, the judgment unit 304 calculates the time TA, which is a time period until a time when 25 g (predetermined amount A1) of the hydrogen peroxide solution has decreased to 1 g (predetermined amount A2), resulting in 24 minutes, for example. Moreover, the judgment unit 307 calculates the time TB, which is a time period until a time when 3 g (predetermined amount A1 + predetermined amount B1) of diluted hydrogen peroxide solution has decreased to 1 g (predetermined amount B2), resulting in 2 minutes. Moreover, the predetermined value in the count unit 306 is assumed to be "4", for example. Thus, in an embodiment of the present invention, the purified water is supplied 4 times.

Moreover, it is assumed that the remaining amount stored in the cup 1100 is zero, the heater 1130 of the sterilization gas generator 1035 is heated, and the carrier gas is supplied to the sterilization gas generator 1035.

First, at time t0, when the operation unit 1024 is operated and the user instructs to generate the hydrogen peroxide gas, the solenoid valve 1032 selects the tank 1030 side (S100, for example). Then, the pump 1033 pumps up 25 g (predetermined amount A1) of the hydrogen peroxide solution from the tank 1030 and supplies it to the sterilization gas generator 1035 (S101, for example). As a result, at time t1 when the supply of the hydrogen peroxide solution is completed, a remaining liquid amount in the cup 1100 of the sterilization gas generator 1035 is 25 g.

Moreover, at the time t1 when the supply of the hydrogen peroxide solution is completed, since the ultrasonic vibrator 1121 is operated (S103, for example), atomization of the hydrogen peroxide solution in the cup 1100 is started. As a result, the atomized hydrogen peroxide solution is heated by the heater 1130, and is supplied as the hydrogen peroxide gas from the sterilization gas generator 1035 to the supply device 1021. Therefore, when the time t1 has passed, the remaining liquid amount of the hydrogen peroxide solution stored in the cup 1100 gradually decreases. At time t2 when 24 minutes (time TA) has elapsed since the time t1, the solenoid valve 1032 selects the tank 1031 side, and the pump 1033 pumps up 2 g (predetermined amount B1) of the purified water from the tank 1031 and supplies it to the cup 1100 (S105, S106, for example). The time t2 is, as described above, a time when it is determined that the remaining liquid amount has reached 1 g (predetermined amount A2) (S104, for example). Thus, at time t3 when the supply of 2 g of the purified water is completed, the remaining liquid amount reaches approximately 3 g. Water feeding performed at the time t2 is the first water feeding.

Since the atomization of the hydrogen peroxide solution still continues at the time t3, the remaining liquid amount in the cup 1100 gradually decreases from the time t3. At time t4 when 2 minutes (time TB) has elapsed since the time t3, as described above, it is determined that the remaining liquid amount has reached 1 g (predetermined amount A2) (S109, for example). Thus, the pump 1033 pumps up 2 g (predetermined amount B1) of the purified water from the tank 1031, and supplies it to the cup 1100 (S106, for example). The water feeding at the time t4 is the second water feeding. In an embodiment of the present invention, operations similar to the operation from the time t2 to the time t4 are repeated from the time t4 until time t8 for the fourth water feeding. When the fourth water feeding is started at the time t8 and completed at time t9, the operation of the ultrasonic vibrator 1121 is ended at time t10 when a predetermined time has elapsed after the time t9, and the atomization is stopped.

The isolator 1010 according to an embodiment of the present invention has been described above. The sterilization gas generator 1035 is configured to generate the hydrogen peroxide gas by atomizing and heating the predetermined amount A1 of the hydrogen peroxide solution, for example. Moreover, when the predetermined amount A1 of the hydrogen peroxide solution is atomized, the concentration of the remaining hydrogen peroxide solution rises. In an embodiment of the present invention, when the judgment unit 304 judges that the predetermined amount A1 of the hydrogen peroxide solution has reached the predetermined amount A2 after atomization of the hydrogen peroxide solution, the valve control unit 300 and the pump control unit 301 supply the purified water to the cup 1100. As a result, the remaining predetermined amount A2 of the hydrogen peroxide solution is diluted, thereby being able to lower the concentration of the hydrogen peroxide solution remaining in the cup 1100. As a result, the atomization of the hydrogen peroxide solution remaining in the cup 1100 is promoted.

Moreover, the valve control unit 300 and the pump control unit 301 is configured to dilute the remaining hydrogen peroxide solution with the purified water in the predetermined amount B1 greater than the predetermined amount A2. Thus, the predetermined amount A2 of the hydrogen peroxide solution is diluted by a factor of at least two or more. Therefore, the concentration of the hydrogen peroxide solution can be reliably lowered.

In general, the diluted hydrogen peroxide solution is atomized more easily than the hydrogen peroxide solution before being diluted. The vibrator control unit 302 according to an embodiment of the present invention is configured to control the ultrasonic vibrator 1121 and keep it operated so as to atomize the diluted hydrogen peroxide solution, as depicted at t3 to t4 in Fig. 16, for example. Thus, the amount of the hydrogen peroxide solution remaining in the sterilization gas generator 1035 can be reduced, thereby being able to prevent deterioration of the sterilizing gas generation device 1035.

Moreover, for example, as in the timing of the time t4 in Fig. 16, when the judgment unit 307 judges that the diluted hydrogen peroxide solution is atomized and has reached the predetermined amount B2, the valve control unit 300 and the pump control unit 301 supply the purified water to the cup 1100. Thus, in an embodiment of the present invention, the hydrogen peroxide solution diluted at the time t2 is diluted again. As a result, in an embodiment of the present invention, the concentration of the hydrogen peroxide solution which deteriorates the sterilization gas generator 1035 can be further lowered.

A description has been given assuming that the predetermined value of the repeat count of the supply of the purified water is 4 in an embodiment of the present invention, but it is not limited thereto, and an arbitrary number of times equal to or greater than 1 may be set. Even if the predetermined value is set at 1, the effect of the present invention can be obtained.

In general, it is possible to a experimentally or theoretically predict a time until when the predetermined amount A1 of the hydrogen peroxide solution is atomized and has decreased to the predetermined amount A2, for example. Thus, when the timer 303 measures the predetermined time TA, the judgment unit 304 judges that the amount of the hydrogen peroxide solution has reached the predetermined amount A2, and when the timer 305 has measured the predetermined time TB, the judgment unit 307 judges that the amount of the diluted hydrogen peroxide solution has reached the predetermined amount B2. As such, in an embodiment of the present invention, the hydrogen peroxide solution can be diluted at desired timing without measuring the amount thereof remaining in the cup 1100 of the sterilization gas generator 1035.

Moreover, for example, if the predetermined amount A1 of the initial hydrogen peroxide solution increases and the predetermined amount A2 of the hydrogen peroxide when diluted decreases, the time TA becomes longer. On the other hand, for example, if the predetermined amount A1 of the initial hydrogen peroxide solution decreases and the predetermined amount A2 of the hydrogen peroxide when diluted increases, the time TA becomes shorter. The time TB is similar to the time TA, and a dilution timing accuracy can be improved by changing the time TA in accordance with the predetermined amounts A1 and A2 as such.

The above-described embodiment is for facilitating understanding of the present invention and is not intended to limit interpretation of the present invention. The present invention can be changed or improved without departing from its gist and also includes the equivalents thereof.

The judgment unit 304 calculates the time TA but it is not limited thereto. For example, a configuration may be such that a time period during which the amount decreases from the predetermined amount A1 to the predetermined amount A2 is actually measured, and information indicating the measurement result is stored as data in the storage device 1070. Then, the determination unit 304 may determine the time TA referring to the data stored in the storage device 1070, on the basis of the information about the predetermined amounts A1 and A2 obtained from the operation result of the operation unit 1024 by the user.

### <<Example 3>>

Subsequently, an example 3 of the present invention will be described.

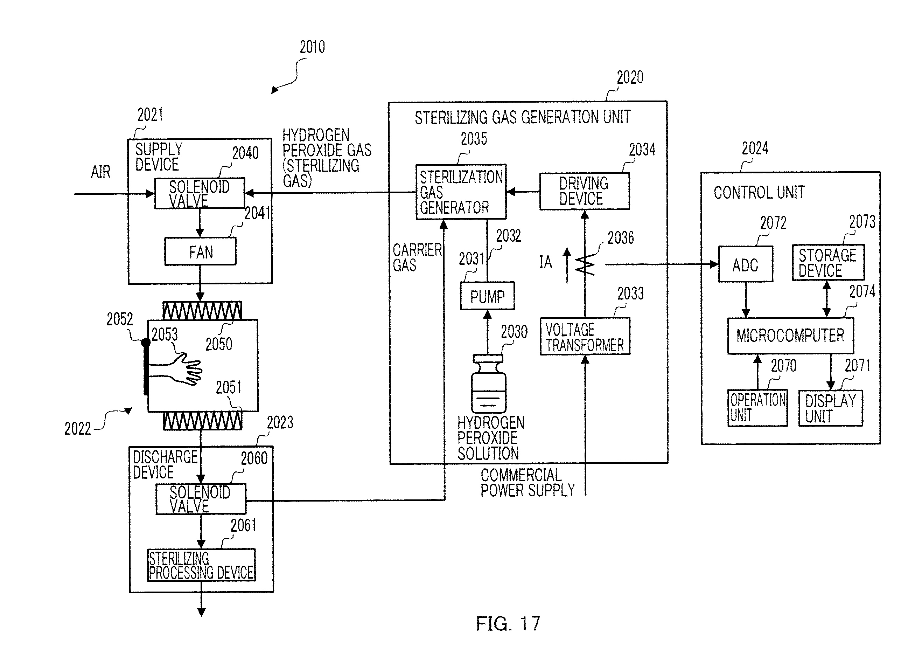

Fig. 17 is a diagram illustrating a configuration of an isolator 2010 according to an embodiment of the present invention. The isolator 2010 is a device for a worker to work on a cell and the like in a sterilized environment, and includes a sterilizing gas generation unit 202020, a supply device 2021, a working chamber 2022, a discharge device 2023, and a control unit 2024.

The sterilizing gas generation unit 2020 is an apparatus unit configured to generate a sterilizing gas using a commercial power supply as a power supply, and includes a tank 2030, a pump 2031, a pipe 2032, a voltage transformer 2033, a driving device 2034, a sterilization gas generator 2035, and a current transformer 2036. Operations of the pump 2031 and the driving device 2034 are controlled by the control unit 2024.

The tank 2030 is configured to store a hydrogen peroxide solution (aqueous solution in which hydrogen peroxide (H₂O₂) is dissolved). The pump 2031 pumps up the hydrogen peroxide solution from the tank 2030, and supplies it to the sterilization gas generator 2035 through the pipe 2032.

The voltage transformer 2033 is configured to transform the commercial power supply voltage and generate a power voltage to operate the driving device 2034.

The driving device 2034 (driving unit) is started when the power supply voltage obtained by being transformed by the voltage transformer 2033 is supplied, and drives the sterilization gas generator 2035 on the basis of an instruction from the control unit 2024. Specifically, the driving device 2034 is configured to vibrate an ultrasonic vibrator, which will be described later, of the sterilization gas generator 2035 when the instruction to generate the sterilizing gas is inputted from the control unit 2024.

The sterilization gas generator 2035 is configured to generate a hydrogen peroxide gas, which is a sterilizing gas, from the supplied hydrogen peroxide solution, and supply the hydrogen peroxide gas to the supply device 2021 together with air, which is a carrier gas. The sterilization gas generator 2035 will be described later in detail.

The current transformer 2036 (measuring unit) is a current transformer configured to measure a current IA supplied from the voltage transformer 2033, which is a power supply of the driving device 2034, to the driving device 2034.

The supply device 2021 is a device configured to supply the supplied hydrogen peroxide gas or air of the exterior of the isolator 2010 to the working chamber 2022, and includes a solenoid valve 2040 and a fan 2041.

The solenoid valve 2040 is configured to supply the hydrogen peroxide gas or the external air to the fan 2041 under control of the control unit 2024. The fan 2041 is configured to supply the hydrogen peroxide gas or the air supplied from the solenoid valve 2040 to the working chamber 2022.

The working chamber 2022 is a space where work on a cell is performed, and the working chamber 2022 is provided with air filters 2050 and 2051, a door 2052, and a working glove 2053.

The air filter 2050 is a filter configured to remove dusts and the like contained in the hydrogen peroxide gas or the air supplied from the fan 2041. The air filter 2051 is a filter configured to remove the dusts and the like contained in a gas or the like discharged from the working chamber 2022. As the air filters 2050 and 2051, an HEPA (High Efficiency Particulate Air) filter is used, for example.

The door 2052 is provided on a front surface of the working chamber 2022 in such a manner as to be capable of being opened/closed, in order to convey a cell and the like to the working chamber 2022.

The working glove 2053 is attached to an opening portion (not shown) provided in the door 2052 so that a worker can work on a cell and the like in the working chamber 2022 in a state where the door 2052 is closed. In a state where the door 2052 is closed, the working chamber 2022 is sealed.

The discharge device 2023 is a device configured to discharge the hydrogen peroxide gas or a gas such as air from the working chamber 2022, and includes a solenoid valve 2060 and a sterilization processing device 2061.

The solenoid valve 2060 is configured to supply a gas outputted from the air filter 2051 to either of the sterilization processing device 2061 or the sterilization gas generator 2035 under control from the control unit 2024. If the output from the solenoid valve 2060 is supplied to the sterilization gas generator 2035, the gas from the working chamber 2022 is circulated.

The sterilization processing device 2061 is provided with a catalyst, and is configured to render the gas outputted from the solenoid valve 2060 harmless and apply sterilization processing thereto, then output it to the exterior of the isolator 2010.

The control unit 2024 is a device configured to control each of the blocks of the isolator 2010, and includes an operation unit 2070, a display unit 2071, an AD converter 2072, a storage device 2073, and a microcomputer 2074.

The operation unit 2070 is an operation panel and the like using which a user set an operation of the isolator 2010. An operation result of the operation unit 2070 is transmitted to the microcomputer 2074.

The display unit 2071 is a display panel configured to display an operation result of the operation unit 2070 and a state and the like of each of the blocks of the isolator 2010.

The AD converter (ADC) 2072 is configured to convert the current IA measured by the current transformer 2036 into digital data.

The storage device 2073 is configured to store program data to be executed by the microcomputer 2074 and various types of data.

The microcomputer 2074 is configured to realize various functions by executing the program data stored in the storage device 2073. For example, when an instruction to generate a sterilizing gas is outputted from the operation unit 2070, the microcomputer 2074 executes a predetermined program for generating the sterilizing gas and controls the pump 2031 and the like. Moreover, the current IA digitized by the AD converter 2072 is inputted to the microcomputer 2074.

The sterilizing gas generation unit 2020 and the control unit 2024 are equivalent to a sterilization substance generator. == Details of sterilization gas generator 2035 ==

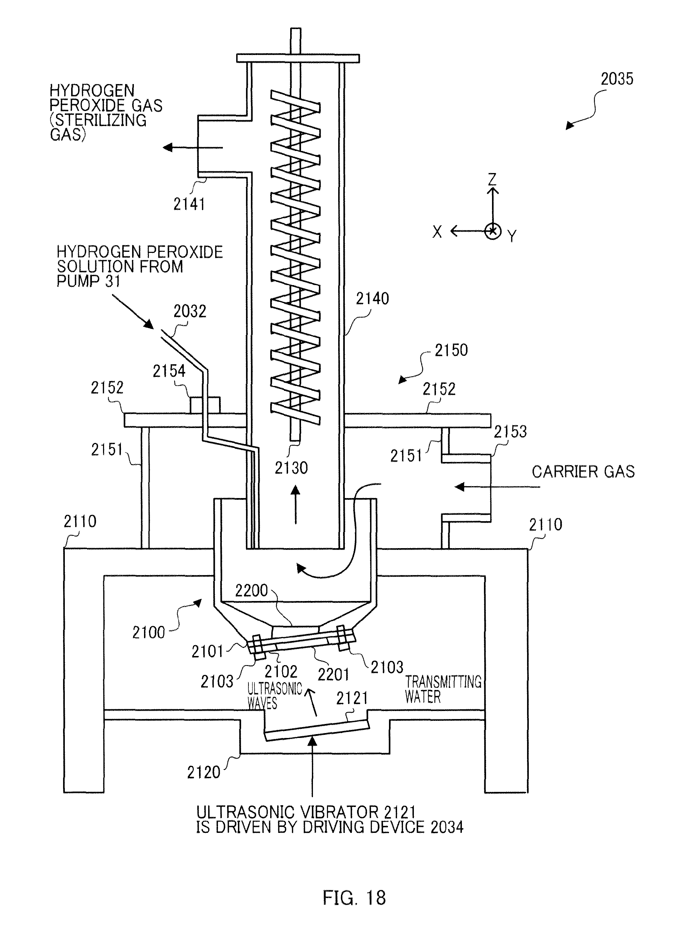

Fig. 18 is a side view of the sterilization gas generator 2035. In Fig. 18, a part of blocks is illustrated in a sectional view. The sterilization gas generator 2035 includes a cup 2100 configured to store the hydrogen peroxide solution and a holding base 2110 configured to hold the cup 2100. An opening portion is provided on an upper side (+Z direction) and a lower side (-Z direction) in the cup 2100. A vibration plate 2101 is fixed to an opening portion 2200 on the lower side of the cup 2100 so as to close the opening portion 2200 using a ring-shaped attaching plate 2102 including an opening portion 2201 and a bolt 2103. The cup 2100, the vibration plate 2101, the attaching plate 2102, and the bolt 2103 are equivalent to a second storage portion.

The interior of the holding base 2110 configured to hold the cup 2100, a partition plate 2120 is attached so as to store the transmitting water for vibrating the vibrating plate 2101 of the cup 2100. Moreover, in the partition plate 2120, an ultrasonic vibrator 2121 configured to vibrate the vibrating plate 2101 with the ultrasonic waves is provided so as to form a predetermined angle with respect to the horizontal direction. That is, the ultrasonic vibrator 2121 is attached to the partition plate 1210 such that a face thereof where the ultrasonic vibrator 2121 generates ultrasonic waves forms a predetermined angle with respect to the X-axis direction.

The vibration plate 2101 on the bottom surface of the cup 2100 is attached such that the vibration plate 2101 is immersed in the transmitting water, and the face where the ultrasonic vibrator 2121 generates the ultrasonic waves and the face of the vibration plate 2101 in the -Z direction are in parallel with each other. That is, the vibration plate 2101 is attached to the cup 2100 such that the face of the vibration plate 2101 in the -Z direction forms a predetermined angle with respect to the X-axis direction.

The ultrasonic vibrator 2121 is configured to generate the ultrasonic waves by being driven by the driving device 2034. Thus, when the ultrasonic waves are supplied to the vibration plate 2101 through the transmitting water, the vibration plate 2101 is vibrated. Then, the hydrogen peroxide solution (liquid) stored in the cup 2100 is atomized. The transmitting water stored in the interior of the holding base 2110 can be replaced through a port (not shown) provided in a side face of the holding base 2110. The holding base 2110 and the partition plate 2120 are equivalent to a first storage portion.

In the upper side of the holding base 2110, a supply pipe 2140 configured to supply the hydrogen peroxide gas to the exterior and a support member 2150 configured to support the supply pipe 2140 are provided. The support member 2150 includes a cylindrical member 2151 mounted on the upper surface of the holding base 2110, and a flange 2152 provided on the upper surface of the cylindrical member 2151.

The cylindrical member 2151 has a diameter greater than the diameter of the cylindrical supply pipe 2140, and a port 2153 supplied with a carrier gas (air circulating in the working chamber 2022) is provided in the side face on the -X side of the cylindrical member 2151. The flange 2152 is configured to close an opening portion on the upper surface of the cylindrical member 2151 and allow the supply pipe 2140 to pass therethrough at the center thereof. The upper surface of the flange 2152 is provided with a port 2154 that is configured to allow the pipe 2032, through which the hydrogen peroxide solution is supplied, to pass therethrough. The pipe 2032 is fixed to the side face of the supply pipe 2140 so that the hydrogen peroxide solution or the like can be supplied to the cup 2100 through the port 2154 and the opening portion provided in the side face of the supply pipe 2140.

Moreover, above the cup 2100, a heater 2130 configured to heat and gasify the atomized hydrogen peroxide solution is provided. The hydrogen peroxide gas heated and gasified by the heater 2130 is outputted from a port 2141 provided in the supply pipe 2140 together with the supplied carrier gas. The port 2141 is connected to the solenoid valve 2040 of the above-described supply device 2021 through a pipe. As such, the hydrogen peroxide solution atomized in the cup 2100 is supplied as the hydrogen peroxide gas from the port 2141 to the supply device 2021. The control unit 2024, the driving device 2034, the current transformer 2036, the cup 2100, the vibrationplate 2101, the attaching plate 2102, the bolt 2103, the holding base 2110, the partition plate 2120, and the ultrasonic vibrator 2121 are equivalent to an atomizing device, and the heater 2130 and the supply pipe 2140 are equivalent to a gasifying unit.

### == Measured waveform of current IA ==

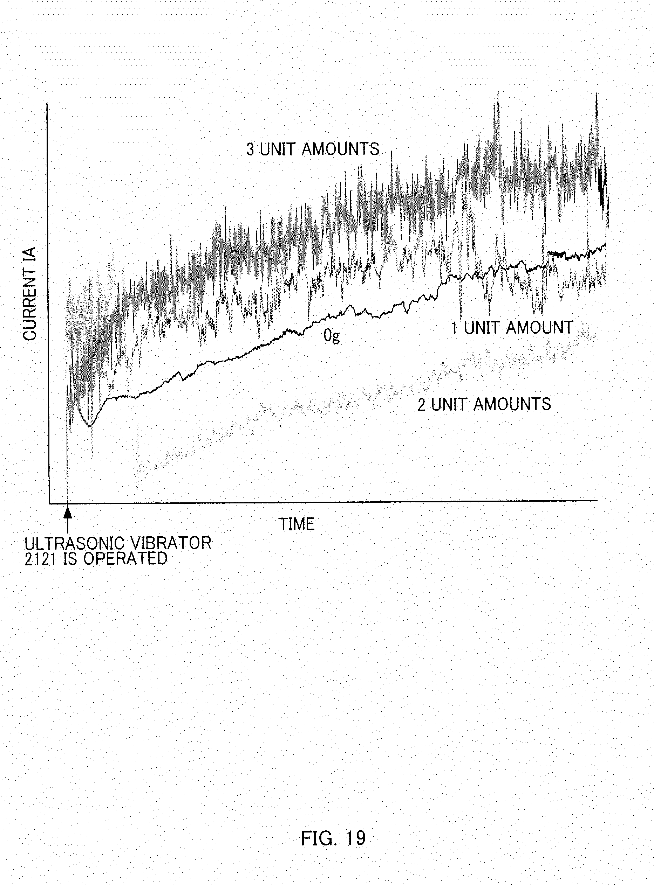

Here, a description will be given, by referring to Fig 19, of a measured waveform of a current value of the current IA when the ultrasonic vibrator 2121 is operated, in a case where purified water (hereinafter simply referred to as water), for example, is poured in advance into the cup 2100 and there is water in the cup 2100, and in a case where there is no water. Here, it is assumed that there is no crack or the like in the vibration plate 2101 attached to the bottom surface of the cup 2100 and no intrusion of the transmitting water into the cup 2100. Fig. 19 illustrates the measured results in the case where each of 1 unit amount, 2 unit amounts, and 3 unit amounts of water is poured into the cup 2100, and in the case where there is no water (0 g).

As obvious from Fig. 19, when an operation of the ultrasonic vibrator 2121 is started, variation in the current IA, that is, fluctuation of the current IA is greater in the case where there is water in the cup 2100 (1 to 3 unit amounts) than that in the case where there is no water (0 g) . Fig. 19 is an experiment result when pouring water into the cup 2100, but even when the hydrogen peroxide solution is poured instead of water, for example, similar waveform is obtained. Moreover, the same applies a case where there is a space between the cup 2100 and the vibration plate 2101 and water intrudes into the cup 2100.

As such, the measured waveform of the current IA while the ultrasonic vibrator 2121 is operated is significantly different depending on presence/absence of water. Therefore, by using the measured result of the current IA, it becomes possible to judge whether or not there is a liquid such as water in the cup 2100.

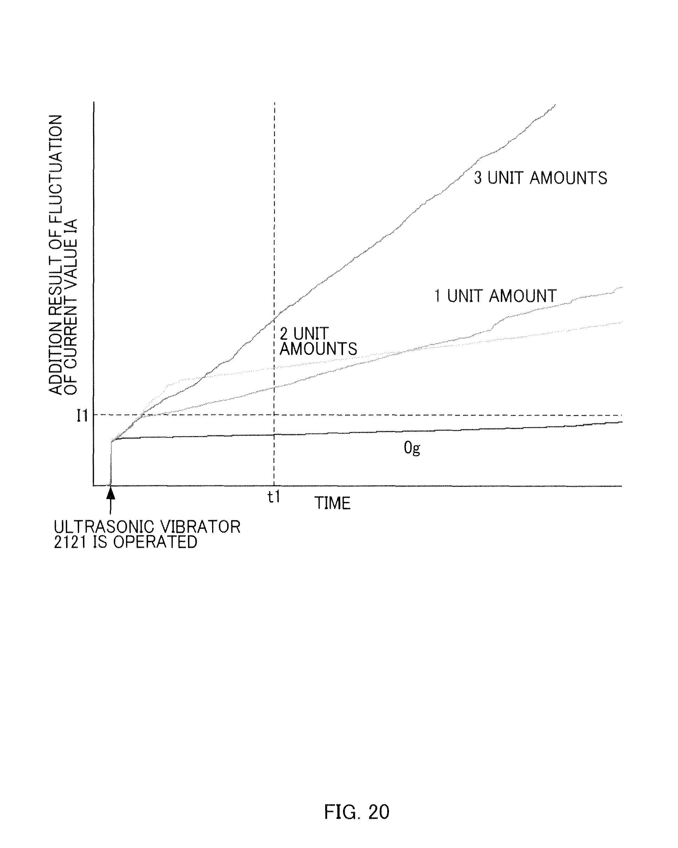

Here, an example of a method of judging presence/absence of a liquid in the cup 2100 will be described using the measured waveform of the current IA, by referring to Fig. 20. Fig. 20 is a result obtained by sequentially adding absolute values of fluctuation of the current IA measured when the water in the cup 2100 is 0 to 3 unit amounts. The fluctuation of the current IA is calculated as a difference between the present current value and a current value of a sample previous thereto when the current IA is sampled and digitized by the AD converter 2072, for example. When adding the fluctuation of the current IA calculated as such, the addition result in the case where there is water in the cup 2100 is greater than the addition result in the case where there is no water.

Moreover, as illustrated in Figs. 19 and 20, the difference between the case where there is water in the cup 2100 and the case there is no water is most remarkable immediately after the vibration of the ultrasonic vibrator 2121 is started, and a predetermined value I1 at timing immediately after the start of vibration may be used. However, though not particularly illustrated, even if there is no water immediately after the start of vibration, the fluctuation of the current IA might become large due to a noise or the like, for example. In this case, in order to suppress the influence of the noise or the like, the influence of the noise may be suppressed by comparing addition results of the cases, at timing later than immediately after the vibration of the ultrasonic vibrator 2121 is started. This will be described by using Figs. 19 and 20. The timing when the noise is suppressed is set at timing when only a predetermined time t1 has elapsed since the start of the vibration of the ultrasonic vibrator 2121, and if the addition result at the timing when only the predetermined time t1 has elapsed after the vibration of the ultrasonic vibrator 2121 is started exceeds a predetermined value I1 which is 1.2 times the addition result in the case where there is no water, for example, it can be determined that there is a liquid such as water in the cup 2100. On the other hand, if the addition result at the predetermined time t1 is smaller than the predetermined value I1, it can be determined that there is no liquid in the cup 2100. The predetermined value I1 is set allowing a margin for the addition result in the case there is no water after the predetermined time t1 has elapsed after the start of vibration. Therefore, if it is known beforehand that the influence of the noise is small, determination time can be reduced by setting the above-described predetermined time t1 at a time immediately after the start of vibration.

### == Details of microcomputer 2074 ==

A description will be given of a functional block realized by the microcomputer 2074 when the hydrogen peroxide gas is generated in the sterilizing gas generation unit 2020.

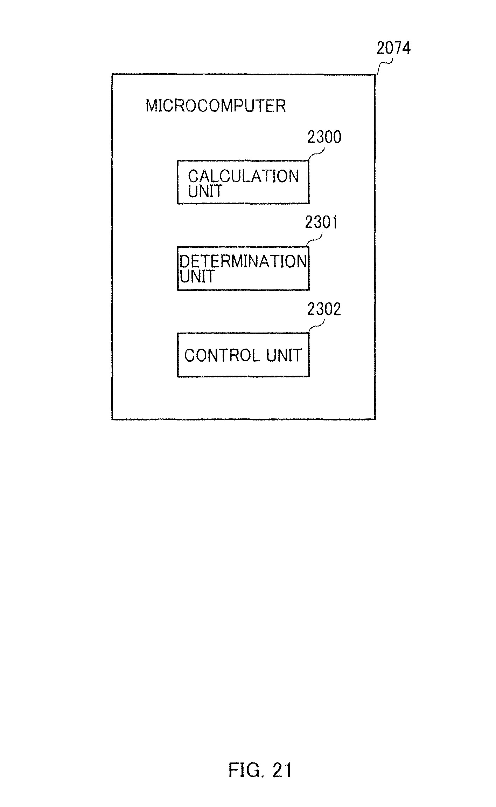

When an instruction to generate the hydrogen peroxide gas is inputted from the operation unit 2070, the microcomputer 2074 executes a predetermined program and realizes functions of a calculation unit 2300, a determination unit 2301, and a control unit 2302 as illustrated in Fig. 21.

The calculation unit 2300 is configured to obtain the digitized current IA sequentially outputted from the AD converter 2072, and calculate a magnitude of the fluctuation of the current IA. Specifically, the calculation unit 2300 is configured to calculate a difference between the present current value and the current value of a sample previous thereto of the current IA. Moreover, the calculation unit 2300 sequentially adds the absolute values of the calculated differences.

The determination unit 2301 is configured to compare the above-described predetermined value I1 and an addition result S1 (value based on the magnitude of the fluctuation of the current IA) of the calculation unit 2300 at the timing when the predetermined time t1 has elapsed since the vibration of the ultrasonic vibrator 2121 is started, that is, the timing when the influence of the noise is suppressed. Then, if the addition result S1 exceeds the predetermined value I1, the determination unit 2301 determines that there is a liquid in the cup 2100. On the other hand, if the addition result S1 does not exceed the predetermined value I1, the determination unit 2301 determines that there is no liquid in the cup 2100. The above-described predetermined value I1 is stored in advance in the storage device 2073, for example.

The control unit 2302 controls the driving device 2034 and drives the ultrasonic vibrator 2121 when an instruction to start processing is inputted from the operation unit 2070. Moreover, if the determination unit 2301 determines that there is no liquid in the cup 2100, the control unit 2302 controls the pump 2031 and the heater 2130 so as to generate the hydrogen peroxide gas. On the other hand, if the determination unit 2301 determines that there is a liquid in the cup 2100, the control unit 2302 causes the display unit 2071 to display that there is a liquid in the cup 2100.

### == Example of processing of microcomputer 2074 ==

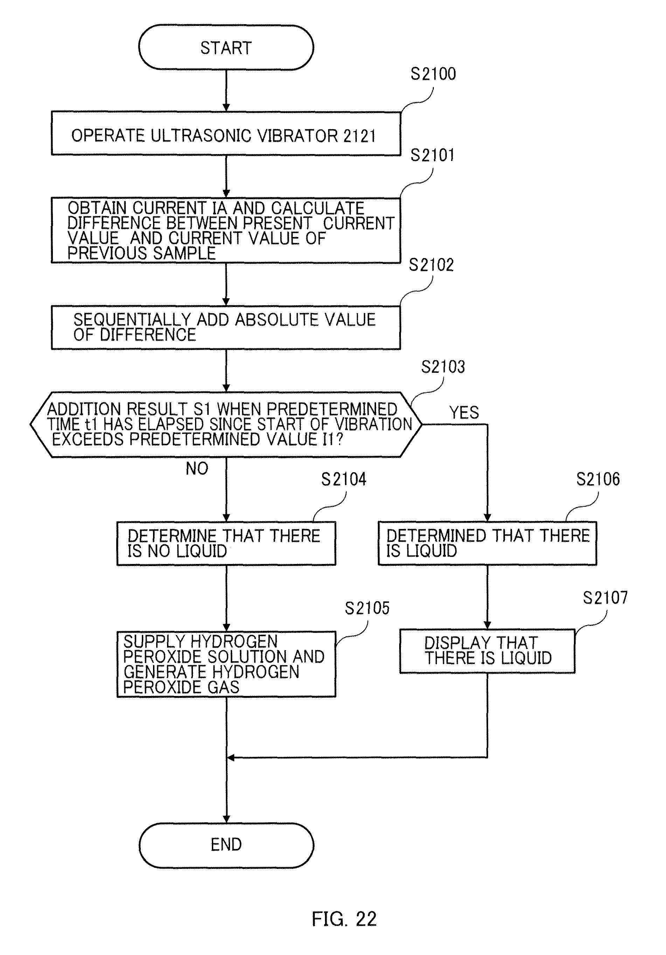

A description will be given, by referring to Fig. 22, of an example of processing executed by the microcomputer 2074 when the hydrogen peroxide gas is generated in the sterilizing gas generation unit 2020.

First, when the operation unit 2070 is operated, and an instruction to generate the hydrogen peroxide gas is inputted to the control unit 2302, the control unit 2302 operates the ultrasonic vibrator 2121 (S2100). Then, the calculation unit 2300 obtains the digitized current IA sequentially outputted from the AD converter 2072, and calculates a difference between the present current value of the current IA and the current value of the current IA of a sample pervious thereto (S2101). Then, the calculation unit 2300 sequentially adds the absolute values of the calculated differences (S2102). Then, the determination unit 2301 compares the predetermined value I1 and the addition result S1 obtained when the predetermined time t1 has elapsed since the start of the vibration of the ultrasonic vibrator 2121 (S2103). Then, when the addition result S1 does not exceed the predetermined value I1 (S2103: NO), the determination unit 2301 determines that there is no liquid in the cup 2100 (S2104). If it is determined that there is no liquid in the cup 2100, the control unit 2302 operates the pump 2031 so that the hydrogen peroxide solution is supplied to the cup 2100, and thereafter causes the heater 2130 to execute heating so that the hydrogen peroxide gas is generated (S2105).

On the other hand, if the addition result S1 exceeds the predetermined value I1 (S2103: YES), the determination unit 2301 determines that there is some liquid in the cup 2100 (S2106). Then, if it is determined that there is a liquid in the cup 2100, the control unit 2302 causes the display unit 2071 to display that there is a liquid in the cup 2100 (S2107).

As described above, the sterilization gas generator 2035 generates the hydrogen peroxide gas only if it is determined that there is no liquid in the cup 2100. On the other hand, if it is determined that there is a liquid in the cup 2100, the display unit 2071 displays as such. Thus, for example, after the user checks the display on the display unit 2071, the user can check whether or not there is a crack or the like in the vibration plate 2101 or there is a space or the like between the cup 2100 and the vibration plate 2101.

Hereinabove, the isolator 2010 according to an embodiment of the present invention has been described. In an embodiment of the present invention, it is determined on the basis of fluctuation of the current IA that there is some liquid in the cup 2100, but it is not limited thereto. As illustrated in Fig. 19, the measured waveform of the current IA is different between the case where there is a liquid in the cup 2100 and the case where there is no liquid. Thus, for example, using the waveform of the current IA when the water is 0 g in Fig. 19 as a reference waveform, presence/absence of the liquid in the cup 2100 may be determined by calculating a difference between the waveform of the current IA measured after the ultrasonic vibrator 2121 is operated and the reference waveform. Specifically, for example, the difference between the measured waveform of the current IA measured when there is no water and the reference waveform is smaller than the difference between the measured waveform of the current IA measured when there is water and the reference waveform. Based on such phenomenon, presence of some liquid in the cup 2100 may be determined by the determination unit 2301.

The case where there is a liquid in the cup 2100 in an embodiment of the present invention can be assumed to be a case where the hydrogen peroxide solution remains when the sterilization gas generator 2035 is operated or a case where a crack or the like appears in the vibration plate 2101 and the transmitting water is intruding into the cup 2100 through the vibration plate 2101, for example. Thus, the determination unit 23 01 can determine whether or not the hydrogen peroxide solution remains in the cup 2100 or whether or not the transmitting water is intruding into the cup 2100 through the vibration plate 2101. As such, in an embodiment of the present invention, whether or not some liquid is present in the cup 2100 can be detected without actually checking the state of the cup 2100.

Moreover, it has become experimentally clear that the magnitude of the fluctuation of the current IA when there is no water in the cup 2100 is smaller in a case where the face at which the ultrasonic vibrator 2121 generates the ultrasonic waves and the bottom surface of the vibration plate 2101 are provided in parallel with each other than that in a case where they are not provided in parallel with each other. Further, it has become also experimentally clear that the magnitude of the fluctuation of the current IA when there is water in the cup 2100 is not greatly changed between the case where the face at which the ultrasonic vibrator 2121 generates the ultrasonic waves and the bottom surface of the vibration plate 2101 are not provided in parallel with each other and other cases. In the sterilization gas generator 2035, the face where the ultrasonic vibrator 2121 generates the ultrasonic waves and the bottom surface of the vibration plate 2101 are provided in parallel with each other. Thus, the difference in the waveform of the current IA between the case where there is no water in the cup 2100 and the case where there is water becomes noticeable. Such a configuration enables more accurate detection of whether or not any liquid is present in the cup 210.

Moreover, the magnitude of the fluctuation of the current IA is changed depending on whether or not there is a liquid in the cup 2100 as illustrated in Fig. 19. Thus, using the calculation unit 2300 and the determination unit 2301 according to an embodiment of the present invention enables determination on whether or not there is a liquid in the cup 2100.

Moreover, as illustrated in Figs. 19 and 20, the case where there is no water and the case where there is water in the cup 2100 can be determined even immediately after the ultrasonic vibrator 2121 starts to vibrate. However, immediately after the start of vibration, the fluctuation of the current IA might become great due to noise or the like, for example. The determination unit 2301 according to an embodiment of the present invention, however, determines in a state where the influence of the noise or the like is suppressed by setting timing after the predetermined time t1 has elapsed since the start of vibration at timing later, in terms of time, than the timing immediately after the start of vibration, and the like. Thus, in an embodiment of the present invention, the influence of the noise or the like can be suppressed, thereby being able to determine presence/absence of a liquid in the cup 2100 with accuracy.

Moreover, if the addition result S1 calculated by the calculation unit 2300 exceeds the predetermined value I1, for example, the determination unit 2301 determines that there is a liquid in the cup 2100. As illustrated in Fig. 19, the magnitude of the fluctuation of the current IA is greater in the case where there is a liquid in the cup 2100 than that in the case where there is no liquid in the cup 2100. Thus, using the calculation unit 2300 and the determination unit 2301 as those in an embodiment of the present invention, presence/absence of the liquid in the cup 2100 can be determined.

The above embodiments of the present invention are simply for facilitating the understanding of the present invention and are not in any way to be construed as limiting the present invention. The present invention may variously be changed or altered without departing from its spirit and encompass equivalents thereof.

For example, in the calculation unit 2300, the difference of the current IA is used as the magnitude of the fluctuation of the current IA, but it is not limited thereto. For example, a standard deviation of the current IA may be used as the magnitude of the fluctuation of the current IA. Specifically, the standard deviation of the current IA in the case where there is a liquid in the cup 2100 is greater than a standard deviation of the current IA in the case where there is no liquid in the cup 2100. Presence/absence of the liquid in the cup 2100 may be determined by the determination unit 2301 in accordance with the magnitude of the standard deviation of the current IA. In such a case, the standard deviation of the current IA acquired when there is no liquid in the cup 2100 in the storage device 2073 is stored and the determination unit 2301 is caused to refer thereto, thereby being able to determine presence/absence of the liquid as in the case with an embodiment of the present invention.

Moreover, for example, the calculation unit 2300 sequentially adds the differences of the currents IA, but an average value of the differences of the currents IA may be calculated, for example. The average value of the differences of the currents IA when there is a liquid in the cup 2100 is greater than the average value of the differences of the currents IA when there is no liquid in the cup 2100. Based on such a calculation result, presence/absence of a liquid in the cup 2100 may be determined by the determination unit 2301.

### REFERENCE SIGNS LIST

- 1: working chamber
- 2: gas supply unit
- 3: gas discharge unit
- 4: sterilizing gas supply device
- 5: control unit
- 6: front-surface door
- 7: working glove
- 8: working space
- 9: gas supply port
- 10: HEPA filter
- 11: gas discharge port
- 12: air inlet
- 13: first three-way valve
- 14: fan
- 15: second three-way valve
- 16: sterilizing substance reduction processing unit
- 17: air outlet
- 18: sterilizing substance cartridge
- 19: pump
- 20: sterilization gas generator
- 21: fan
- 30: atomizing unit
- 31: housing unit
- 32: ultrasonic vibrator
- 33: storage portion
- 34: vibration plate
- 35: ultrasonic transmitting liquid
- 36: partition plate
- 37: housing body
- 38: concave portion
- 39: O-ring
- 40: fixing screw
- 41: upper end portion of storage portion
- 50: gasifying unit
- 51: internal cylindrical portion
- 52: heater
- 53: external cylindrical portion
- 54: lower end portion of external cylindrical portion
- 55: lower end portion of internal cylindrical portion
- 56: heat generating body
- 57: fin
- 58: flow path regulating plate
- 59: piping
- 60: upper end portion of internal cylindrical portion
- 61: internal lid member
- 62: piping
- 63: gas flow path
- 64: tube opening
- 65: upper end portion of external cylindrical portion
- 66: external lid member
- 70: external flow path regulating plate
- 71: preheating heater
- 72: preheating heater
- 73: heat generating body
- 74: fin
- 75: flow path regulating plate
- 1010: isolator
- 1020: sterilizing gas generation unit
- 1021: supply device
- 1022: working chamber
- 1023: discharge device
- 1024: operation unit
- 1025: control unit
- 1030,: 1031 tank
- 1032, 1040, 1060: solenoid valve
- 1033: pump
- 1034: pipe
- 1035: sterilization gas generator
- 1041: fan
- 1050, 1051: air filter
- 1052: door
- 1053: glove
- 1061: sterilization processing device
- 1070: storage device
- 1071: microcomputer
- 1100: cup
- 1101: vibration plate
- 1102: attaching plate
- 1103: bolt
- 1110: holding base
- 1120: partition plate
- 1121: ultrasonic vibrator
- 1130: heater
- 1140: supply pipe
- 1141, 1153, 1154: port
- 1150: support member
- 1151: cylindrical member
- 1152: flange
- 1200, 1201: opening portion
- 300: valve control unit
- 301: pump control unit
- 302: vibrator control unit
- 303, 305: timer
- 304, 307: judgment unit
- 306: count unit
- 10: isolator
- 2020: sterilizing gas generation unit
- 2021: supply device
- 2022: working chamber
- 2023: discharge device
- 2024: control unit
- 2030: tank
- 2031: pump
- 2032: pipe
- 2033: voltage transformer
- 2034: driving device
- 2035: sterilization gas generator
- 2036: current transformer
- 2040, 60: solenoid valve
- 2041: fan
- 2050, 51: air filter
- 2052: door
- 2053: glove
- 2061: sterilization processing device
- 2070: operation unit
- 2071: display unit
- 2072: AD converter
- 2073: storage device
- 2074: microcomputer
- 2100: cup
- 2101: vibration plate
- 2102: attaching plate
- 2103: bolt
- 2110: holding base
- 2120: partition plate
- 2121: ultrasonic vibrator
- 2130: heater
- 2140: supply pipe
- 2141, 153, 154: port
- 2150: support member
- 2151: cylindrical member
- 2152: flange
- 2200, 201: opening portion
- 2300: calculation unit
- 2301: determination unit
- 2302: control unit

## Claims

1. A hydrogen peroxide gas generator comprising:
an atomizing unit configured to atomize hydrogen peroxide stored in a storage portion by applying ultrasonic vibration;
a heater provided above the atomizing unit, the heater configured to heat and gasify the hydrogen peroxide atomized in the atomizing unit;
an internal cylindrical portion, made of metal, whose internal space has the heater arranged therein, the internal cylindrical portion configured to guide upward the hydrogen peroxide atomized in the atomizing unit flowing together with a carrier gas; and
an external cylindrical portion, double-pipe constructed, whose internal space has the internal cylindrical portion arranged therein, having a gas flow path for the carrier gas flowing downward toward the storage portion formed between the external cylindrical portion and the internal cylindrical portion,
the carrier gas flowing through the gas flow path caused to contact the internal cylindrical portion heated by the heater, the heated carrier gas introduced to the storage portion.

2. The hydrogen peroxide gas generator according to claim 1, wherein
the storage portion includes a concave portion concave in an inverted truncated conical shape, and
the internal cylindrical portion is configured with a cylindrical member having a lower end portion cut diagonally with respect to a longitudinal direction of a cylinder, and the lower end portion has a tip portion arranged at a height close to an inclined surface of the concave portion so that a flow velocity of the carrier gas passing along the tip portion of the lower end portion becomes higher than a flow velocity of the carrier gas passing along other portions of the lower end portion.

3. The hydrogen peroxide gas generator according to one of claims 1 and 2, wherein
an internal fin, made of a metal plate having thermal conductivity, is configured to regulate a flow direction of the atomized hydrogen peroxide, the internal fin attached between an internal wall surface of the internal cylindrical portion and the heater.

4. The hydrogen peroxide gas generator according to one of claims 1 to 3, wherein
an external fin, made of a metal plate having thermal conductivity, is configured to regulate a flow direction of the carrier gas, the external fin attached between an external wall surface of the internal cylindrical portion and an internal wall surface of the external cylindrical portion.

5. The hydrogen peroxide gas generator according to one of claims 1 to 4, wherein
the external cylindrical pipe is made of a metal material having thermal conductivity lower than thermal conductivity of the internal cylindrical pipe.

6. The hydrogen peroxide gas generator according to one of claims 1 to 5, wherein
a preheating heater configured to preheat the carrier gas is provided at some midpoint in a gas introduction pipe configured to introduce the carrier gas into the gas flow path.

7. A sterilization substance generator comprising:
an atomizing unit including a storage portion configured to store hydrogen peroxide solution, and an ultrasonic vibrator configured to atomize the hydrogen peroxide solution by applying ultrasonic vibration to the hydrogen peroxide solution stored in the storage portion;
a gasifying unit configured to heat and gasify the hydrogen peroxide solution atomized by the atomizing unit, and output the gasified solution together with a supplied carrier gas;
a first supply unit configured to supply the hydrogen peroxide solution to the storage portion;
a second supply unit configured to supply a diluent to the storage portion;
a determination unit configured to determine whether or not an amount of the hydrogen peroxide solution remaining in the storage portion has reached a first predetermined amount since atomization of the hydrogen peroxide solution stored in the storage portion, based on a supply amount of the hydrogen peroxide solution supplied from the first supply unit to the storage portion; and
a control unit configured to
control the atomizing unit so as to atomize the hydrogen peroxide solution stored in the storage portion, and
control the second supply unit so as to supply the diluent to the storage portion when the determination unit has determined that an amount of the hydrogen peroxide solution remaining in the storage portion has reached the first predetermined amount.

8. The sterilization substance generator according to claim 7, wherein
the control unit is configured to control the second supply unit so as to supply, to the storage portion, an amount of the diluent greater than the first predetermined amount.

9. The sterilization substance generator according to one of claims 7 and 8, wherein
the control unit is configured to control the atomizing unit so as to atomize the diluted hydrogen peroxide solution acquired after the diluent is supplied to the storage portion.

10. The sterilization substance generator according to claim 9, wherein
the determination unit is configured to determine whether or not the amount of the diluted hydrogen peroxide solution remaining in the storage portion has reached a second predetermined amount since atomization of the diluted hydrogen peroxide solution stored in the storage portion, based on an amount of the diluted hydrogen peroxide solution when the diluent has been supplied to the storage portion; and
the control unit is configured to control the second supply unit so as to supply the diluent to the storage portion when the determination unit has determined that an amount of the diluted hydrogen peroxide solution remaining in the storage portion has reached the second predetermined amount.

11. The sterilization substance generator according to claim 10, wherein
the determination unit includes:
a first timer configured to start measuring time, when the hydrogen peroxide solution has been supplied from the first supply unit;
a second timer configured to start measuring time, when the diluent has been supplied from the second supply unit; and
a judgment unit configured to judge that the hydrogen peroxide solution has reached the first predetermined amount when the first timer has measured a first time period, and judge that the diluted hydrogen peroxide solution has reached the second predetermined amount when the second timer has measured a second time period.

12. The sterilization substance generator according to claim 11, wherein
the first time period is a time period according to the supply amount and the first predetermined amount, and
the second time period is a time period according to the second predetermined amount and an amount of the diluted hydrogen peroxide solution acquired when the diluent has been supplied to the storage portion.

13. An atomizing device comprising:
a first storage portion configured to store transmitting water;
a second storage portion, configured to store liquid, provided such that a bottom surface attached with a vibration plate is immersed in the transmitting water;
an ultrasonic vibrator configured to apply ultrasonic vibration to the vibration plate through the transmitting water and atomize the liquid, the ultrasonic vibrator mounted on a bottom surface of the first storage portion such that a face of the ultrasonic vibrator where ultrasonic waves are generated forms a predetermined angle with respect to a horizontal direction;
a driving unit configured to drive the ultrasonic vibrator so as to generate ultrasonic waves at the ultrasonic vibrator;
a measuring unit configured to measure a current supplied to the driving unit from a power supply supplied to the driving unit; and
a determination unit configured to determine whether or not the liquid remains in the second storage portion or the transmitting water has intruded into the second storage portion through the vibration plate, based on a measurement result of the measuring unit.

14. The atomizing device according to claim 13, wherein
the vibration plate is attached to the second storage portion so as to be substantially parallel with the face of the ultrasonic vibrator where the ultrasonic waves are generated.

15. The atomizing device according to one of claims 13 and 14, further comprising:
a calculation unit configured to calculate a magnitude of fluctuation of the current based on a measurement result of the measuring unit, wherein
the determination unit is configured to determine whether or not the liquid remains in the second storage portion or the transmitting water has intruded into the second storage portion through the vibration plate, based on a magnitude of the fluctuation of the current calculated by the calculation unit.

16. The atomizing device according to claim 15, wherein
the determination unit is configured to determine whether or not the liquid remains in the second storage portion or the transmitting water has intruded into the second storage portion through the vibration plate, based on a magnitude of the fluctuation of the current when a predetermined time period has elapsed after the driving unit starts to drive the ultrasonic vibrator.

17. The atomizing device according to one of claims 15 and 16, wherein
the determination unit is configured to determine that the liquid remains in the second storage portion or the transmitting water has intruded into the second storage portion through the vibration plate, when a value acquired based on a magnitude of the fluctuation of the current exceeds a predetermined value.

18. A sterilizing substance generator, comprising
a first storage portion configured to store transmitting water;
a second storage portion, configured to store a hydrogen peroxide solution, provided such that a bottom surface attached with a vibration plate is immersed in the transmitting water;
an ultrasonic vibrator configured to apply ultrasonic vibration to the vibration plate through the transmitting water and atomize the liquid, the ultrasonic vibrator mounted on a bottom surface of the first storage portion such that a face of the ultrasonic vibrator where ultrasonic waves are generated forms a predetermined angle with respect to a horizontal direction;
a gasifying unit configured to heat and gasify the atomized hydrogen peroxide solution, and output the gasified solution together with a supplied carrier gas;
a driving unit configured to drive the ultrasonic vibrator so as to generate ultrasonic waves at the ultrasonic vibrator;
a measuring unit configured to measure a current supplied to the driving unit from a power supply supplied to the driving unit; and
a determination unit configured to determine whether or not the hydrogen peroxide solution remains in the second storage portion or the transmitting water has intruded into the second storage portion through the vibration plate, based on a measurement result of the measuring unit.
